# EUROPEAN PATENT APPLICATION

(11) **EP 4 834 303 A1**
(43) Date of publication of application: **04.11.2026**
(21) Application number: 24911920.7
(22) Date of filing: 08.10.2024
(51) Int. Cl.: A61H 3/04, A61B 5/00, A61B 5/11, A61B 5/16, A61B 5/107, A61B 5/389, A61G 5/04, G06T 7/00, G06T 7/20, G10L 15/10

(54) **FUNCTION IMPROVEMENT ASSISTANCE DEVICE AND FUNCTION IMPROVEMENT ASSISTANCE METHOD**

(30) Priority: 28.12.2023 JP 2023223726
(71) Applicant: CYBERDYNE INC., Tsukuba-shi, Ibaraki 305-0818 (JP)
(72) Inventor: SANKAI, Yoshiyuki, Tsukuba-shi, Ibaraki 305-0818 (JP)
(74) Representative: Mewburn Ellis LLP
(86) International application number: PCT/JP2024/036037
(87) International publication number: WO 2025/142026

(57) **Abstract**

A current state of physical functions and cognitive functions of a subject is determined based on human information including brain and nervous system information, muscular and skeletal system information, physiological information, psychological information, action information, and behavior information of the subject, and while a normal force at a contact portion with the subject with respect to a state holding variable mechanism unit is measured in real time, mechanism switching, a transformation state, and a power application state of the state holding variable mechanism unit are controlled based on those determination result and measurement result.

## Description

### Technical Field

The present invention relates to a function improvement support apparatus and a function improvement support method, and is particularly suitable for application to improve functions of daily life in a subject who has been bedridden for a relatively long time.

### Background Art

Conventionally, when physical strength weakens and a bedridden state occurs once, frailty (muscle weakness) due to the bedridden state and a decline in cognitive ability are accelerated, and it has been very difficult to lead an independent daily life from a stage at which the bedridden state has occurred.

These bedridden patients wear diapers all day long, which makes it very difficult for them to perform daily activities such as going to the toilet and defecating on their own. In fact, the problem is that the patient can only see the ceiling and is very likely to become hyper delirious and in a state of complete and severe physical and cognitive care, making it very difficult for the patient to live an independent daily life, such as shopping, walking, and communicating.

To overcome such a problem, conventionally, a walking assist chair that can be used as both a wheelchair and a walker has been proposed to improve the mobility and walking ability of bedridden people (see Patent Literature 1).

In the walking assist chair in Patent Literature 1, when used as a wheelchair, a wheelchair seat surface is formed by a central lifting seat surface and a pair of left and right retractable seat surfaces, whereas when used as a walker, only the lifting seat surface is raised while the pair of retractable seat surfaces are retracted, so that a user can walk while applying body weight to the lifting seat surface by simply slightly lowering the hips.

The walking assist chair is also designed to reduce the force with which the user pushes the walking assist chair by moving the vehicle portion in its walking direction in accordance with the user's movements when the chair is used as a walker.

A further development of this walking assist chair has been proposed by the same inventor as in Patent Literature 1 (see Patent Literature 2). In the walking assist chair in Patent Literature 2, in addition to operating as both an electric wheelchair and a walking assist device, when shifting from the electric wheelchair to the walking assist device, the walking assist chair operates as a standing assist device that assists standing of a user seated in the electric wheelchair, whereas when shifting from the walking assist device to the electric wheelchair, the walking assist chair operates as a seating assist device that assists seating of the user who is walking with the walking assist device onto the electric wheelchair.

### Citation List

### Patent Literature

Patent Literature 1: Japanese Patent Laid-Open No. 2013-85716
Patent Literature 2: Japanese Patent Laid-Open No. 2019-208561

### Summary of Invention

### Technical Problem

However, in each of the walking assist chairs in Patent Literatures 1 and 2, transition from an electric wheelchair to a walking assist device and transition from a walking assist device to an electric wheelchair are performed based on an operation of a user, and except for following the user in accordance with a walking speed of the user, almost all operations are merely assisted in accordance with the operation of the user.

Therefore, in these walking assist chairs, a target user is limited to a person having weakened legs and hips who requires a general wheelchair, and it is very difficult for a person having a relatively low level of independence, such as a person in a bedridden state, to actively perform operations for movement and walking by themself.

In order to improve mobility and walking ability in these relatively less independent individuals, it is necessary to improve physical and neurological functions by linking tricuspid and visual information with physical functions through repetition of standing and seated postures and walking movements.

However, the walking assist chairs of Patent Literatures 1 and 2 mentioned above are insufficient in practical use to improve the physical and neurological functions of bedridden people and improve their activities of daily living (ADL) and quality of life (QOL).

The present invention was made in consideration of the above points, and attempts to propose a function improvement support apparatus and a function improvement support method that can significantly improve the degree of independence in daily life and realize combined functional improvement of the subject's physical, psychological, and social aspects.

### Solution to Problem

In order to solve such problems, the present invention includes a posture holding variable mechanism unit having a wheelchair function that holds a subject in a sitting posture and travels in accordance with an operation by the subject, and a walking assist function that holds the subject in a standing posture and applies an assist force in accordance with a walking motion of the subject, the posture holding variable mechanism unit being transformable, while holding the subject, from a first mechanism corresponding to the wheelchair function to a second mechanism corresponding to the walking assist function, or from the second mechanism to the first mechanism, a human information acquisition unit that acquires human information including brain and nervous system information, muscular and skeletal system information, physiological information, psychological information, action information, and behavior information of the subject, a current state function determination unit that determines a current state of physical functions and cognitive functions of the subject based on the human information obtained by the human information acquisition unit, a normal force measurement unit provided in the state holding variable mechanism unit and configured to measure, in real time, a normal force at a contact portion with the subject, and a control unit that controls mechanism switching, a transformation state, and a power application state of the state holding variable mechanism unit based on the determination result by the current state function determination unit and the measurement result by the normal force measurement unit.

As a result, in the function improvement support apparatus, safe movement in a wide space is performed in a sitting state by the wheelchair function, and, as necessary, daily life in a standing state or a walking state in a living space is enabled by the walking assist function. In particular, subjects who have been bedridden for a relatively long time can improve their physical and neurological functions by repeatedly performing sitting and standing postures in conjunction with tricuspid and visual information and physical functions, which can significantly improve their independence in daily living.

According to the present invention, the control unit detects, based on the measurement result of the normal force at the contact portion with the subject by the normal force measurement unit, a transition of a center of gravity position of the subject and a load distribution to a specific portion at the contact portion, determines a tensed state and a relaxed state of the subject at a time of a sitting posture, a standing posture, and a transition to both postures of the subject while detecting the transition of the center of gravity position and the load distribution, and then adjusts a control content of the state holding variable mechanism unit in accordance with the tensed state and the relaxed state.

As a result, in the function improvement support apparatus, while a tensed state and a relaxed state are determined at a time of transition of a posture of the subject, by adjusting control relating to the mechanism switching, the transformation state, and the power application state of a state holding variable mechanism unit, guidance from a sitting state to a standing state of the subject and guidance from a standing state to a sitting state of the subject can be performed more safely.

Furthermore, the present invention further includes an environment information acquisition unit that acquires environment information representing a surrounding environment of the subject and a floor surface state on which the state holding variable mechanism unit moves, and the control unit, when controlling the mechanism switching, the transformation state, and the power application state of the state holding variable mechanism unit, prioritizes ensuring safety of the subject based on the environment information obtained from the environment information acquisition unit.

As a result, in the function improvement support apparatus, while a surrounding floor surface state and a bodily sensation state are recognized, safety of traveling and walking of the subject is ensured and a risk of falling can be significantly reduced.

The present invention further includes a wheel drive unit provided in the posture holding variable mechanism unit and configured to independently drive a pair of left and right drive wheels common to the first and second mechanisms, an imaging unit that captures a lower body of the subject and sequentially acquires an RGB image and a depth image, an image coordinate setting unit that, in a state in which the posture holding variable mechanism unit is transformed into the second mechanism corresponding to the wheelchair function, sets, as image coordinates, a plurality of skeletal representative points attached to a lower body of the subject while estimating a posture of the lower body of the subject based on the RGB images sequentially acquired from the imaging unit, a coordinate group extraction unit that sequentially extracts, from among the image coordinate groups set by the image coordinate setting unit, image coordinate groups related to a gait state of the subject based on a transition state of a posture of the subject, a skeletal information acquisition unit that acquires temporally continuous three-dimensional skeletal information centered on a lower body of the subject by time-synchronizing the image coordinate groups sequentially extracted by the coordinate group extraction unit with the depth images sequentially acquired from the imaging unit, and a gait state detection unit that detects, based on the three-dimensional skeletal information sequentially acquired by the skeletal information acquisition unit, a gait state that is a connection of a plurality of local actions from a transition state of a posture of the subject, and the control unit generates power synchronized with the gait state detected by the gait state detection unit to the wheel drive unit.

As a result, in the function improvement support apparatus, a gait state of the subject can be detected in real time, and a walking motion can be assisted while being synchronized with the gait state.

The present invention further includes a wheel drive unit provided in the posture holding variable mechanism unit and configured to independently drive a pair of left and right drive wheels common to the first and second mechanisms, a biological signal detection unit that is arranged on a body surface portion of the subject and has an electrode group for detecting a biological signal of the subject, an action intent recognition unit that, in a state in which the posture holding variable mechanism unit is transformed into the first mechanism corresponding to the wheelchair function, recognizes an action intent of the subject with respect to a specific operation content among operation contents in the wheelchair function based on the biological signal obtained from the biological signal detection unit, and an operation content understanding unit that understands an operation content that is an action intent of the subject recognized by the action intent recognition unit, and the control unit generates power to the wheel drive unit in accordance with the operation content understood by the operation content understanding unit.

As a result, in the function improvement support apparatus, during a wheelchair function, traveling can be performed without the subject operating the apparatus by themself while reflecting an operation content corresponding to an action intent of the subject.

The present invention further includes a gaze detection unit that, while capturing an image of a space corresponding to a visual field of the subject, recognizes, by image recognition, a movement of an eyeball of the subject within an imaging range and detects a position of a gaze of the subject, and the action intent recognition unit recognizes, based on the position of the gaze detected by the gaze detection unit, that the position of the gaze is a movement target as an action intent of the subject when the position of the gaze continues for a predetermined time or longer.

As a result, in the function improvement support apparatus, not only a biological signal of the subject but also a position of a gaze is used to recognize an action intent of the subject with respect to an operation content, whereby traveling can be performed while reflecting an operation content corresponding to the action intent of the subject more effectively.

The present invention further includes a sound collection unit that collects a voice of the subject, and an utterance content recognition unit that recognizes an utterance content based on the voice collected by the sound collection unit, and the action intent recognition unit recognizes, based on the utterance content recognized by the utterance content recognition unit, an operation content corresponding to the utterance content as an action intent of the subject.

As a result, in the function improvement support apparatus, not only a biological signal of the subject but also an utterance content of the subject is used to recognize an action intent of the subject with respect to an operation content, whereby traveling can be performed while reflecting an operation content corresponding to the action intent of the subject more effectively.

Furthermore, the present invention, in which a posture holding variable mechanism unit having a wheelchair function that holds a subject in a sitting posture and travels in accordance with an operation by the subject, and a walking assist function that holds the subject in a standing posture and applies an assist force in accordance with a walking motion of the subject, is configured to be transformable, while holding the subject, from a first mechanism corresponding to the wheelchair function to a second mechanism corresponding to the walking assist function, or from the second mechanism to the first mechanism, includes a first step of acquiring human information including brain and nervous system information, muscular and skeletal system information, physiological information, psychological information, action information, and behavior information of the subject, a second step of determining a current state of physical functions and cognitive functions of the subject based on the human information obtained by the first step, a third step of measuring, in real time, a normal force at a contact portion with the subject with respect to the state holding variable mechanism unit, and a fourth step of controlling mechanism switching, a transformation state, and a power application state of the state holding variable mechanism unit based on a determination result by the second step and a measurement result by the third step.

As a result, in the function improvement support apparatus, safe movement in a wide space is performed in a sitting state by the wheelchair function, and, as necessary, daily life in a standing state or a walking state in a living space is enabled by the walking assist function. In particular, subjects who have been bedridden for a relatively long time can improve their physical and neurological functions by repeatedly performing sitting and standing postures in conjunction with tricuspid and visual information and physical functions, which can significantly improve their independence in daily living.

### Advantageous Effects of Invention

According to the present invention, it is possible to provide a function improvement support apparatus and a function improvement support method capable of significantly improving a degree of independence in daily life and realizing combined functional improvement of physical, psychological, and social aspects of a subject.

### Brief Description of Drawings

[Figure 1] Figure 1 is a conceptual diagram illustrating an external configuration of a function improvement support apparatus according to an embodiment.
[Figure 2] Figure 2 is a conceptual diagram illustrating a configuration of the posture holding variable mechanism unit shown in Figure 1.
[Figure 3] Figure 3 is a conceptual diagram illustrating a circuit configuration of a control system in the function improvement support apparatus shown in Figure 1.
[Figure 4] Figure 4 is a conceptual diagram for explaining a human information acquisition unit.
[Figure 5] Figure 5 is a conceptual diagram illustrating a state in which the posture holding variable mechanism unit is the first mechanism corresponding to the wheelchair function.
[Figure 6] Figure 6 is a conceptual diagram illustrating a state in which the posture holding variable mechanism unit is the second mechanism corresponding to the walking assist function.
[Figure 7] Figure 7 is a conceptual diagram for explaining mutual transformation between the wheelchair function and the walking assist function.
[Figure 8] Figure 8 is a perspective view and a six-sided view showing an external configuration of a vital sign measurement apparatus.
[Figure 9] Figure 9 is a conceptual diagram illustrating a configuration of a brain activity measurement unit.
[Figure 10] Figure 10 is a conceptual diagram illustrating a configuration of a biological signal measurement wearing device.
[Figure 11] Figure 11 is a conceptual diagram for explaining a worn state of the biological signal measurement wearing device.
[Figure 12] Figure 12 is a conceptual diagram illustrating an additional configuration of the vital sign measurement apparatus.
[Figure 13] Figure 13 is a graph showing pulse waveforms before and after filter application.
[Figure 14] Figure 14 is a conceptual diagram representing an ROI of a near-infrared image and an ROI of a far-infrared image.
[Figure 15] Figure 15 is a conceptual diagram for explaining nasal breathing and mouth breathing.
[Figure 16] Figure 16 is a conceptual diagram for explaining temperature states of nasal breathing and mouth breathing.
[Figure 17] Figure 17 is a graph representing changes in nasal temperature due to nasal breathing and changes in oral temperature due to mouth breathing.
[Figure 18] Figure 18 is a graph in which a change in breathing method from nasal breathing to mouth breathing and state information of each state are superimposed.
[Figure 19] Figure 19 is a conceptual diagram for explaining non-contact measurement of oxygen saturation using light.
[Figure 20] Figure 20 is a block diagram illustrating an internal configuration of a motion capture device.
[Figure 21] Figure 21 is a conceptual diagram for explaining a method for acquiring three-dimensional skeletal information of a whole body of a subject.
[Figure 22] Figure 22 is a conceptual diagram representing a structure of a behavior recognition model.
[Figure 23] Figure 23 is a conceptual diagram for explaining hybrid control during wheelchair function.
[Figure 24] Figure 24 is a conceptual diagram illustrating another embodiment of the posture holding variable mechanism unit.

### Description of Embodiments

An embodiment of the present invention is described in detail below with reference to drawings.

### (1) Configuration of Function Improvement Support Apparatus According to Embodiment

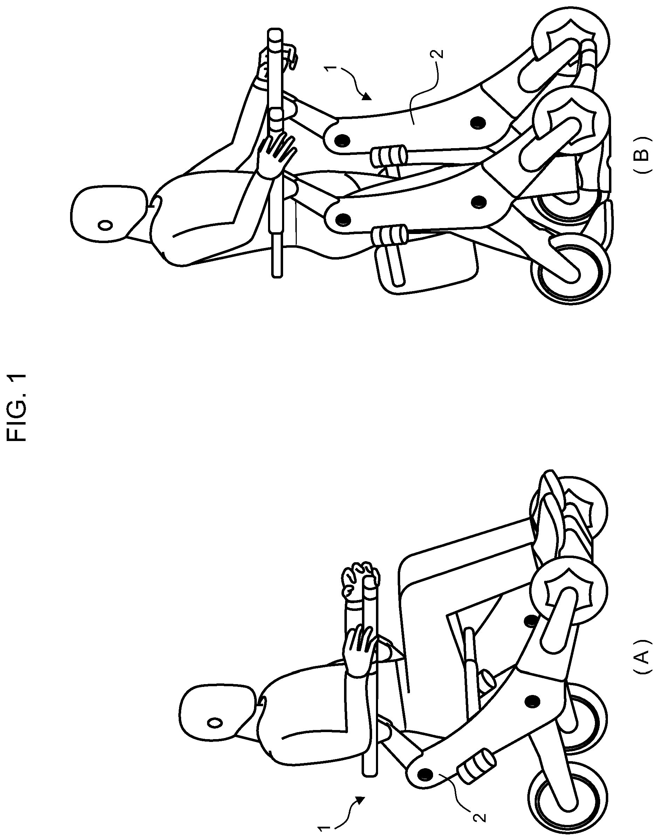

Figures 1(A) and 1(B) show a function improvement support apparatus 1 according to this embodiment. The function improvement support apparatus 1 has both a wheelchair function (Figure 1(A)) that holds a subject in a sitting posture and travels in accordance with an operation by the subject, and a walking assist function (Figure 1(B)) that holds the subject in a standing posture and applies an assist force in accordance with a walking motion of the subject.

The function improvement support apparatus 1 has a posture holding variable mechanism unit 2 that is switchable, in accordance with an action of a subject, between a first mechanism (Figure 1(A)) corresponding to a wheelchair function and a second mechanism (Figure 1(B)) corresponding to a walking assist function. The posture holding variable mechanism unit 2 is configured to be transformable, while holding the subject, from a first mechanism corresponding to a wheelchair function to a second mechanism corresponding to a walking assist function, or from the second mechanism to the first mechanism.

### (2) Configuration of Posture Holding Variable Mechanism Unit

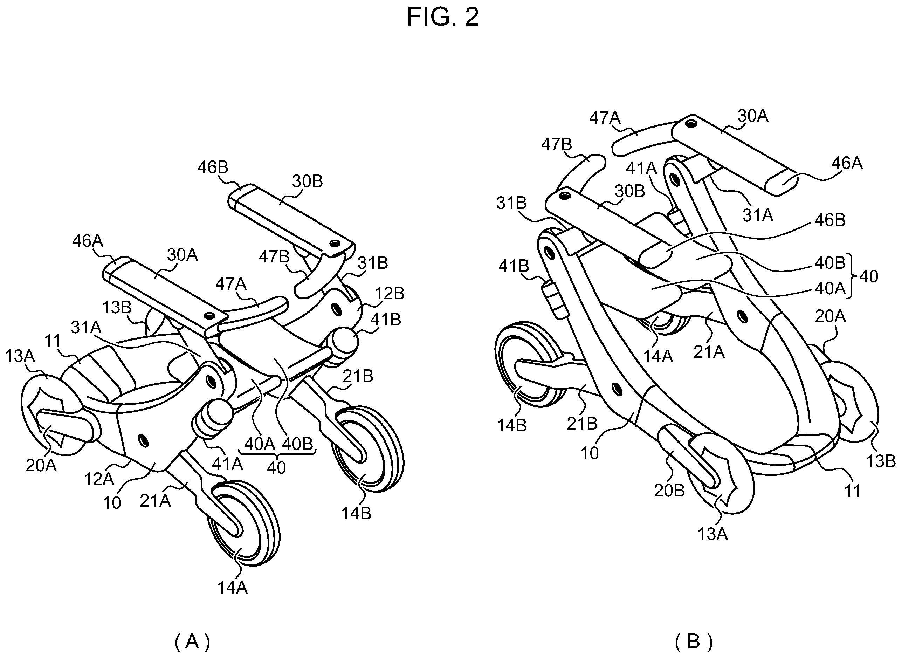

As shown in Figures 2(A) and 2(B), the posture holding variable mechanism unit 2 has a frame structure body 10 having a generally U-shaped and left-right symmetrical structure as a whole, and the frame structure body 10 is composed of a foot placement portion 11 having a footrest function at a curved center and a left frame 12A and a right frame 12B that are left-right symmetrical with respect to the foot placement portion 11.

The left frame 12A and the right frame 12B in the frame structure body 10 each have a shape bent at a predetermined angle around a middle portion along a longitudinal direction.

In addition, in the left frame 12A and the right frame 12B, one end of front-side arm portions 20A and 20B, in which front wheel portions 13A and 13B are respectively provided, is fixed in a vicinity of the foot placement portion 11, and one end of rear-side arm portions 21A and 21B, in which rear wheel portions 14A and 14B are respectively provided, is movably provided at bent portions.

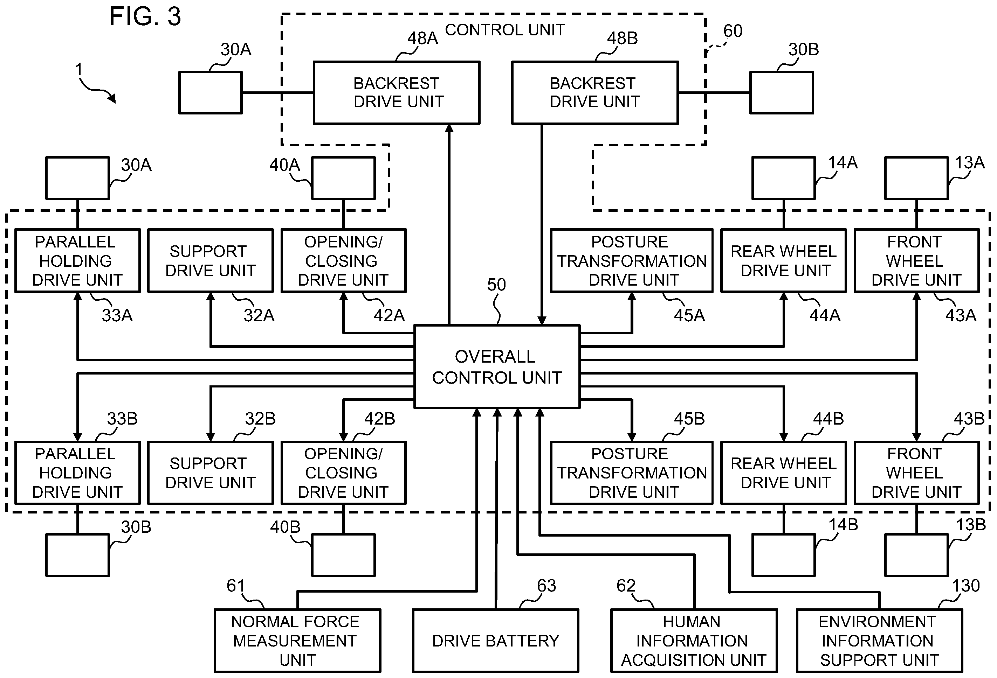

Further, in the left frame 12A and the right frame 12B, at end portions, the other end of support arm portions 31A and 31B, to one end of which armrest portions 30A and 30B are respectively attached, is movably provided. The left and right support arm portions 31A and 31B are engaged integrally with corresponding drive mechanism systems ( support drive units 32A and 32B and parallel holding drive units 33A and 33B shown in Figure 3 described later), respectively.

Further, in the left frame 12A and the right frame 12B, at predetermined portions between the support arm portions 31A and 31B and the rear-side arm portions 21A and 21B, fixed holding portions 41A and 41B, to which a part of a seat portion 40 is movably attached, are respectively provided. The left and right fixed holding portions 41A and 41B are engaged with corresponding opening/closing drive units 42A and 42B (Figure 3), respectively.

The left and right front wheel portions 13A and 13B are composed of drive wheels having a pair of left and right omni-wheel structures (a wheel structure in which a plurality of rollers are rotatably attached so as to be orthogonal to an axle, and the rollers are arranged in a circumferential direction so as to match a curvature of a circle centered on the axle), the drive wheels being rotatable in a traveling direction or in a direction intersecting the traveling direction in accordance with traveling of an apparatus main body.

The left and right front wheel portions 13A and 13B are engaged with corresponding front wheel drive units 43A and 43B (Figure 3) through the front-side arm portions 20A and 20B, respectively. The front wheel drive units 43A and 43B drive and rotate, about a drive shaft provided at the other end of the front-side arm portions 20A and 20B as a rotation center, the corresponding front wheel portions 13A and 13B in a traveling direction and in a direction intersecting the traveling direction, respectively.

The left and right rear-side arm portions 21A and 21B are each connected to rear wheel portions 14A and 14B at the other ends and are engaged integrally with corresponding drive mechanism systems (rear wheel drive units 44A and 44B and posture transformation drive units 45A and 45B shown in Figure 3 described later), respectively. The rear wheel drive units 44A and 44B drive and rotate, about a drive shaft provided at the other end of the rear-side arm portions 21A and 21B as a rotation center, the corresponding rear wheel portions 14A and 14B.

The posture transformation drive units 45A and 45B (Figure 3) drive and rotate, about rotation shafts provided at the respective bent portions of the left frame 12A and the right frame 12B described above as rotation centers, one ends of the rear-side arm portions 21A and 21B, respectively. The posture transformation drive units 45A and 45B incorporate a lock mechanism for suppressing rotation (see Japanese Patent Nos. 4997416 and 5344501 by the present inventor), and are configured to ensure safety of the subject by preventing rotation of one ends of the left and right rear-side arm portions 21A and 21B in a direction opposite to a driving direction.

The support drive units 32A and 32B (Figure 3) drive and rotate, about rotation shafts provided at end portions of the left frame 12A and the right frame 12B described above as rotation centers, the other ends of the support arm portions 31A and 31B, respectively. The parallel holding drive units 33A and 33B (Figure 3) drive and rotate, about one end of the left and right support arm portions 31A and 31B as rotation centers, the armrest portions 30A and 30B, respectively.

The left and right armrest portions 30A and 30B each have a flat surface for placing an upper arm of a subject, and controllers 46A and 46B for operation by the subject are respectively provided at front ends in a longitudinal direction, and curved frame bodies 47A and 47B corresponding to a backrest of the subject are respectively rotatably attached at rear ends in the longitudinal direction.

The left and right curved frame bodies 47A and 47B are in a pair relationship, and are designed in advance such that, in a closed state (a state in which front ends face each other), a form in which a waist portion of the subject is held from a back side (a backrest forming state) is obtained. Backrest drive units 48A and 48B (Figure 3) are incorporated in the left and right armrest portions 30A and 30B, and the backrest drive units 48A and 48B are configured to drive a pair of curved frame bodies 47A and 47B to open and close simultaneously.

The support drive units 32A and 32B, the parallel holding drive units 33A and 33B, and the backrest drive units 48A and 48B are configured to be driven in conjunction with each other under control of an overall control unit 50 described later so that flat surfaces of the left and right armrest portions 30A and 30B are maintained in a substantially horizontal state with respect to a floor surface that is always a traveling surface.

The seat portion 40 is configured to form a seating surface by being combined into one from a state in which it is divided into left and right halves. The seat portion 40 is provided such that a left seat half body 40A corresponding to the left frame 12A and a right seat half body 40B corresponding to the right frame 12B are each rotatably provided within a predetermined angle range about the fixed holding portions 41A and 41B as rotation references.

The opening/closing drive units 42A and 42B (Figure 3) rotate, within a predetermined angle range, a left seat half body 40A and a right seat half body 40B constituting the seat portion 40 about the fixed holding portions 41A and 41B provided in the left frame 12A and the right frame 12B described above as rotation centers, thereby forming the seat portion 40 of the subject, and are opened so as to be in a substantially parallel relationship with the left frame 12A and the right frame 12B.

The opening/closing drive units 42A and 42B are configured to be driven, in accordance with control of the overall control unit 50, so as to adjust rotation angles of a left seat half body 40A and a right seat half body 40B constituting the seat portion 40 while operating in conjunction with the posture transformation drive units 45A and 45B.

In the function improvement support apparatus 1, in the posture holding variable mechanism unit 2, a control unit 60 is provided inside a foot placement portion 11 of a frame structure body 10, normal force measurement units 61 are provided on flat surfaces of the left and right armrest portions 30A and 30B and on respective seating surfaces of a left seat half body 40A and a right seat half body 40B, and a human information acquisition unit 62 is attached to a part of the posture holding variable mechanism unit 2 and to the subject (Figure 3). Further, in the posture holding variable mechanism unit 2, an environment information acquisition unit 130 is provided as described later, so that a subject can recognize a surrounding floor surface state and a bodily sensation state.

Figure 3 shows a circuit configuration of a control system in the function improvement support apparatus 1. In the function improvement support apparatus 1, an overall control unit 50 including an MCM (Multi-Chip Module) having a CPU (Central Processing Unit) and a memory is incorporated in the control unit 60 in the posture holding variable mechanism unit 2, and is configured to collectively control the support drive units 32A and 32B, the parallel holding drive units 33A and 33B, the opening/closing drive units 42A and 42B, the front wheel drive units 43A and 43B, the rear wheel drive units 44A and 44B, the posture transformation drive units 45A and 45B, and the backrest drive units 48A and 48B.

The support drive units 32A and 32B, the parallel holding drive units 33A and 33B, the opening/closing drive units 42A and 42B, the front wheel drive units 43A and 43B, the rear wheel drive units 44A and 44B, the posture transformation drive units 45A and 45B, and the backrest drive units 48A and 48B each have a drive actuator (not shown), and are configured to drive the respective drive actuators and transmit output in accordance with control by the overall control unit 50.

Further, the function improvement support apparatus 1 incorporates, in the frame structure body 10, a relatively large-capacity drive battery 63 including a secondary battery or a capacitor, and is capable of supplying power to drive actuators of the support drive units 32A and 32B, the parallel holding drive units 33A and 33B, the opening/closing drive units 42A and 42B, the front wheel drive units 43A and 43B, the rear wheel drive units 44A and 44B, the posture transformation drive units 45A and 45B, and the backrest drive units 48A and 48B.

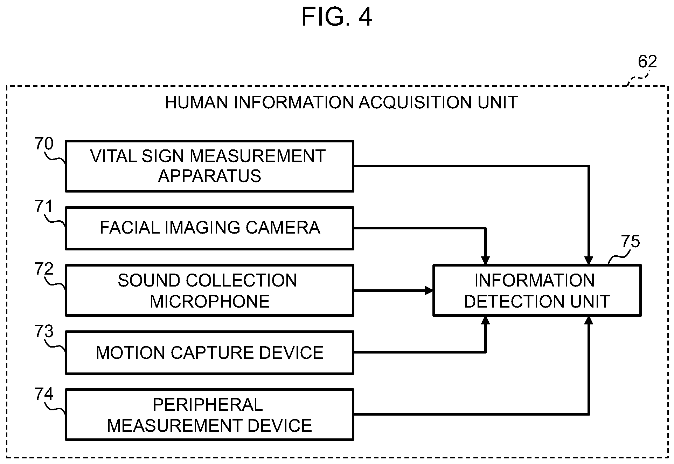

As shown in Figure 4, the human information acquisition unit 62 acquires human information including brain and nervous system information, muscular and skeletal system information, physiological information, psychological information, action information, and behavior information of a subject. Specifically, the human information acquisition unit 62 has a vital sign measurement apparatus 70 (measurement means for electrocardiogram, pulse, pulse pressure, oxygen saturation, body temperature, electroencephalogram, blood glucose level, blood pressure, and electromyogram), a face imaging camera 71, a sound collection microphone 72, a motion capture device 73 (optical type, inertial sensor type, mechanical type, magnetic type, or a combination thereof), peripheral measurement devices 74 (thermometers, hygrometers, ambient sound collectors for audible sound and ultrasonic waves, etc.), and an information detection unit 75 that detects human information described later based on outputs of these sensor groups and devices.

In the human information acquisition unit 62, the information detection unit 75 detects brain and nervous system information, muscular and skeletal system information, and physiological information of a subject based on detection results by the vital sign measurement apparatus 70, and detects psychological information by analyzing a facial expression and a voice of the subject using the face imaging camera 71 and the sound collection microphone 72, and further detects action information and behavior information of the subject by the motion capture device 73.

The overall control unit 50 has a role as a current state function determination unit, and determines a current state of physical functions and cognitive functions of a subject based on human information obtained by the human information acquisition unit 62.

The normal force measurement unit 61 is incorporated in a seat portion 40 and armrest portions 30A and 30B of the state holding variable mechanism unit 2, and measures, in real time, a normal force at a contact portion with a subject. Specifically, the normal force measurement unit 61 has a structure in which a plurality of strain gauges (strain detection elements) are respectively arranged in a plane on the seat portion 40 and the armrest portions 30A and 30B in a predetermined group arrangement, and is configured to measure all or a part of external forces in three-dimensional directions acting on contact portions of the subject.

As the plurality of strain gauges constituting the normal force measurement unit 61, in order to detect a minute strain change with high sensitivity, strain detection elements in which a piezo resistor is formed by partially doping impurities into a thin plate-shaped silicon substrate, and strain detection elements in which a metal thin film is formed on a silicon substrate are used.

The normal force measurement unit 61 divides contact portions of a subject into a plurality of parts in mesh units based on output signals from respective strain gauges, and analyzes stress at each node of elements to analyze stress and strain of the contact portions (finite element method). As a result, the normal force measurement unit 61 measures, in real time, three-dimensional external forces acting on contact portions with a subject as a normal force based on a distribution state and a transition state of the analyzed stress and strain.

The overall control unit 50 comprehensively determines brain and nervous system information, muscular and skeletal system information, and physiological information of a subject, psychological information of the subject, and action information and behavior information of the subject based on human information obtained from the human information acquisition unit 62, and determines a current state of physical functions (a degree of comparison for each element with respect to reference physical functions) and cognitive functions (a degree of comparison with respect to a predetermined cognitive determination criterion) of the subject.

### (2-1) Modes of Wheelchair Function and Walking Assist Function

In the function improvement support apparatus 1, the posture holding variable mechanism unit 2 is configured such that a first mechanism (Figure 1(A)) corresponding to a wheelchair function that holds a subject in a sitting posture and travels in accordance with an operation by the subject, and a second mechanism (Figure 1(B)) corresponding to a walking assist function that holds the subject in a standing posture and applies an assist force in accordance with a walking motion of the subject are mutually transformable while holding the subject.

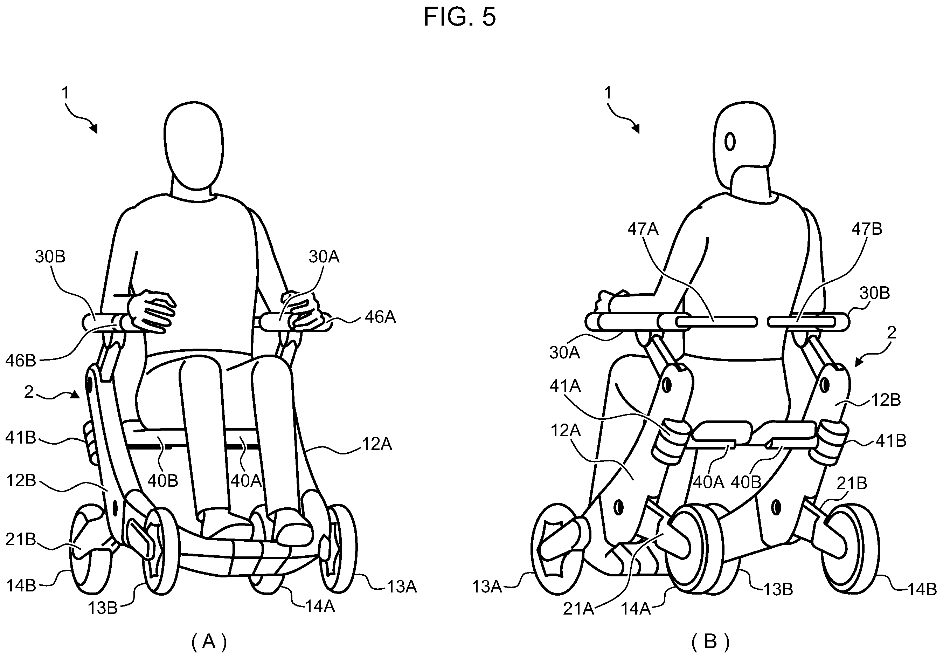

Figures 5(A) and 5(B) show a state in which the posture holding variable mechanism unit 2 is the first mechanism corresponding to the wheelchair function. In the first mechanism, the rear-side arm portions 21A and 21B are respectively positioned with respect to the left frame 12A and the right frame 12B such that the rear wheel portions 14A and 14B are located farthest from the front wheel portions 13A and 13B.

In the first mechanism, the left seat half body 40A and the right seat half body 40B engaged with the fixed holding portions 41A and 41B form the seat portion 40 such that seating surfaces are substantially horizontal, and the pair of curved frame bodies 47A and 47B are positioned so as to be in a backrest forming state while flat surfaces of the armrest portions 30A and 30B are maintained substantially horizontal.

Thus, in the function improvement support apparatus 1, by setting the posture holding variable mechanism unit 2 to a state of the first mechanism corresponding to the wheelchair function, a subject, while being seated on the seat portion 40, places both arms on the armrest portions 30A and 30B and places both feet on the foot placement portion 11, and is enabled to perform traveling operation using the controllers 46A and 46B provided at front ends of the armrest portions 30A and 30B while maintaining a sitting posture equivalent to that of a wheelchair.

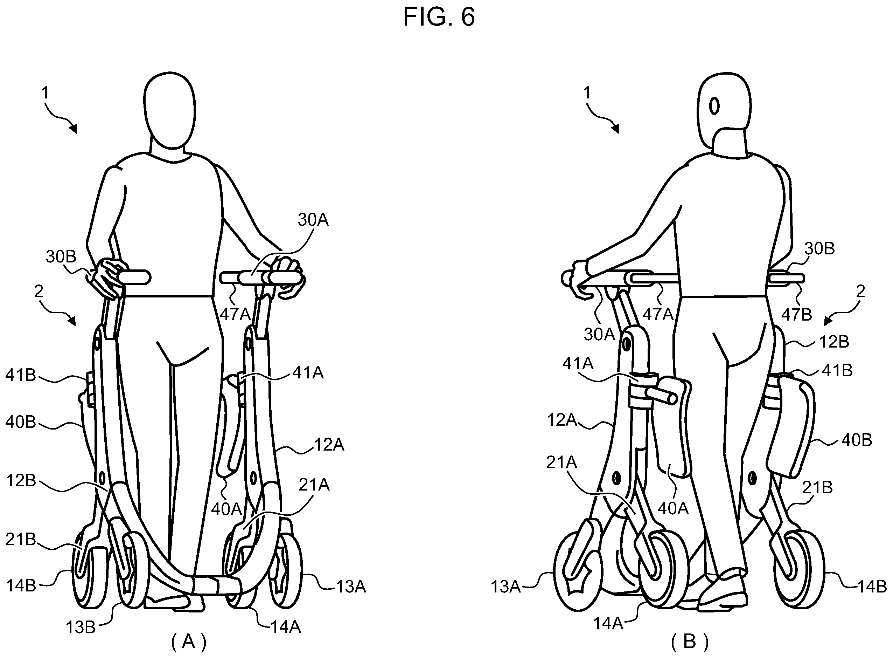

On the other hand, Figures 6(A) and 6(B) show a state in which the posture holding variable mechanism unit 2 is the second mechanism corresponding to the walking assist function. In the second mechanism, the rear-side arm portions 21A and 21B are respectively positioned with respect to the left frame 12A and the right frame 12B such that the rear wheel portions 14A and 14B are located closest to the front wheel portions 13A and 13B.

In the second mechanism, the left seat half body 40A and the right seat half body 40B engaged with the fixed holding portions 41A and 41B are positioned such that seating surfaces are in a substantially parallel relationship with the left frame 12A and the right frame 12B, and the pair of curved frame bodies 47A and 47B are positioned so as to be in an open state while flat surfaces of the armrest portions 30A and 30B are maintained substantially horizontal.

Thus, in the function improvement support apparatus 1, by setting the posture holding variable mechanism unit 2 to a state of the second mechanism corresponding to the walking assist function, a subject is enabled to walk in a standing posture while grasping the left and right armrest portions 30A and 30B and while applying an assist force in accordance with a walking motion.

### (2-2) Mutual Transformation between Wheelchair Function and Walking Assist Function

In the function improvement support apparatus 1, the posture holding variable mechanism unit 2 is configured such that it can be transformed, while holding a subject, from a first mechanism corresponding to a wheelchair function to a second mechanism corresponding to a walking assist function, or from the second mechanism to the first mechanism.

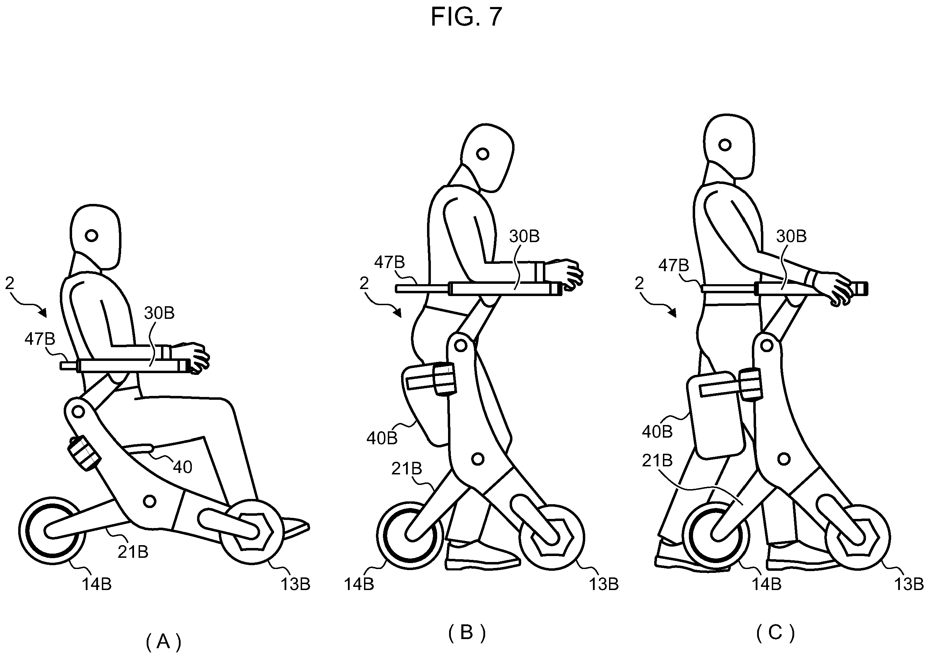

In the posture holding variable mechanism unit 2, in a state of the first mechanism, the overall control unit 50 determines, while causing the subject to travel in a desired direction in accordance with an operation of the subject, whether the subject starts transition from a sitting posture to a standing posture based on a determination result as a current state function determination unit and a real-time measurement result of a normal force applied to the seat portion 40 and the armrest portions 30A and 30B by the normal force measurement unit 61 (Figure 7(A)).

Then, when the subject starts transition to a standing posture, the overall control unit 50 controls the posture transformation drive units 45A and 45B to start rotating the rear-side arm portions 21A and 21B such that the rear wheel portions 14A and 14B approach the front wheel portions 13A and 13B.

Subsequently, as the subject transitions from a sitting posture to a standing posture, the overall control unit 50 controls the opening/closing drive units 42A and 42B to start opening the left seat half body 40A and the right seat half body 40B constituting the seat portion 40, and at the same time controls the backrest drive units 48A and 48B to also start opening the pair of curved frame bodies 47A and 47B. At this time, the overall control unit 50 controls the parallel holding drive units 33A and 33B such that flat surfaces of the left and right armrest portions 30A and 30B are each always maintained in a horizontal state (Figure 7(B)).

The overall control unit 50 causes the posture holding variable mechanism units 45A and 45B to be in a state of the second mechanism described above when the subject has completely transitioned to a standing posture. That is, the overall control unit 50 controls the posture transformation drive units 45A and 45B to position the rear wheel portions 14A and 14B at positions closest to the front wheel portions 13A and 13B, and at the same time positions the left seat half body 40A and the right seat half body 40B such that seating surfaces are in a substantially parallel relationship with the left frame 12A and the right frame 12B, and positions the pair of curved frame bodies 47A and 47B such that they are in a fully open state (Figure 7(C)).

On the other hand, in the posture holding variable mechanism unit 2, in a state of the second mechanism, the overall control unit 50 determines, while performing an assist operation in synchronization with a walking motion of the subject, whether the subject starts transition from a standing posture to a sitting posture based on a determination result as a current state function determination unit and a real-time measurement result of a normal force applied to the seat portion 40 and the armrest portions 30A and 30B by the normal force measurement unit 61 (Figure 7(C)).

Then, when the subject starts transition to a sitting posture, the overall control unit 50 controls the posture transformation drive units 45A and 45B to start rotating the rear-side arm portions 21A and 21B such that the rear wheel portions 14A and 14B move away from the front wheel portions 13A and 13B.

Subsequently, as the subject transitions from a standing posture to a sitting posture, the overall control unit 50 controls the opening/closing drive units 42A and 42B to start forming the seat portion 40 by causing the left seat half body 40A and the right seat half body 40B to face each other, and at the same time controls the backrest drive units 48A and 48B to also start causing the pair of curved frame bodies 47A and 47B to face each other to be in a backrest forming state. At this time, the overall control unit 50 controls the parallel holding drive units 33A and 33B such that flat surfaces of the left and right armrest portions 30A and 30B are each always maintained in a horizontal state (Figure 7(B)).

The overall control unit 50 causes the posture holding variable mechanism unit 2 to be in a state of the first mechanism described above when the subject has completely transitioned to a sitting posture. That is, the overall control unit 50 controls the posture transformation drive units 45A and 45B to position the rear wheel portions 14A and 14B at positions farthest from the front wheel portions 13A and 13B, and at the same time forms the seat portion 40 having substantially horizontal seating surfaces by causing the left seat half body 40A and the right seat half body 40B to face each other, and positions the pair of curved frame bodies 47A and 47B such that they are in a backrest forming state (Figure 7(A)).

The overall control unit 50 controls, when transforming from the first mechanism to the second mechanism or from the second mechanism to the first mechanism while holding the subject, mechanism switching, a transformation state, and a power application state of the state holding variable mechanism unit 2 based on a determination result as a current state function determination unit and a measurement result by the normal force measurement unit 61.

That is, the overall control unit 50 controls, based on a current state of physical functions and cognitive functions of a subject as a determination result of a current state function determination unit and a normal force applied to the seat portion 40 and the armrest portions 30A and 30B as a measurement result by the normal force measurement unit 61, switching of the state holding variable mechanism unit 2 from a state of a first mechanism corresponding to a wheelchair function or a second mechanism corresponding to a walking assist function, controls a transformation state in a process of the mechanism switching (a speed of transformation, a posture position, and mechanism switching in a reverse direction according to a situation), and also controls a power application state in the transformation state.

As a result, in the function improvement support apparatus 1, safe movement in a wide space is performed in a sitting state by the wheelchair function, and, as necessary, daily life in a standing state or a walking state in a living space is enabled by the walking assist function. In particular, subjects who have been bedridden for a relatively long time can improve their physical and neurological functions by repeatedly performing sitting and standing postures in conjunction with tricuspid and visual information and physical functions, which can significantly improve their independence in daily living.

### (3) Detailed Configuration and Functions of Human Information Acquisition Unit

### (3-1) Configuration of Vital Sign Measurement Apparatus

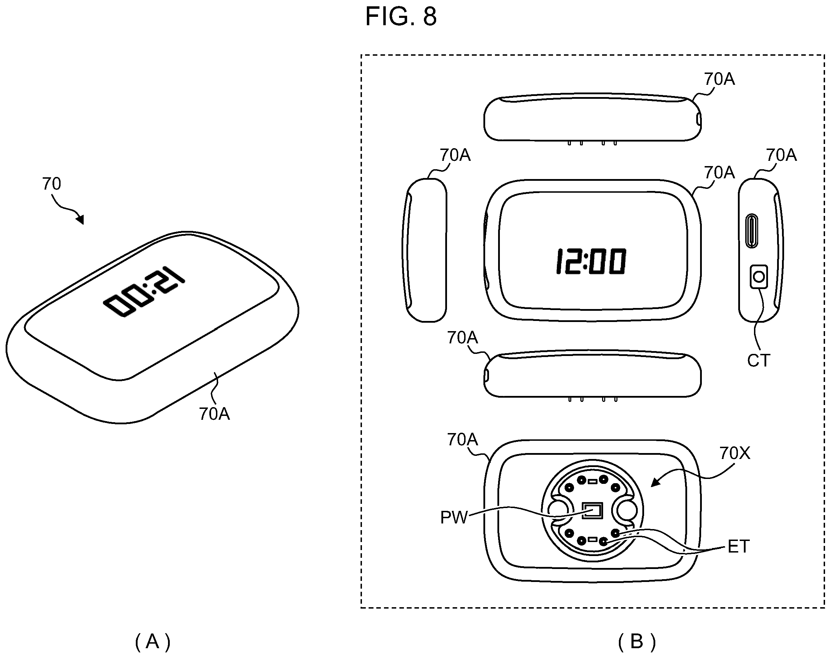

Specifically, as shown in Figure 8(A), the vital sign measurement apparatus 70 has an electrode terminal group ET and a photoelectric probe window PW arranged in a predetermined pattern on a measurement surface 70X of the device body 70A and is configured to measure the subject's biological information through the measurement surface 70X. The biometric information covered by the vital sign measurement apparatus 70 is a measurement result of at least one or more of electrocardiogram, pulse rate, pulse pressure, oxygen saturation, body temperature, electroencephalogram, blood glucose level, blood pressure, and myoelectricity. In other words, the vital sign measurement apparatus 70 is configured so that the same device body can be used as means for measuring all types of biometric information.

As shown in Figure 8(B), the vital sign measurement apparatus 70 has a sound collection microphone 72 (Figure 4) detachably connected to a connection terminal CT provided on a side of the device body 70A, which can collect vibrations generated from the subject's larynx and pharynx as acoustic signals when connected. As a result, it becomes possible to acquire, for the subject, information on vibrations (non-audible range) associated with the heart and respiration that cannot be heard by the ear.

### (3-2) Detection method of brain/nervous system information

The human information acquisition unit 62 has, as an additional configuration of the vital sign measurement apparatus 70, a brain activity measurement unit 80 that is attached to the subject's head and measures electroencephalograms and cerebral blood flow in a measurement region of the head. The information detection unit 75 then converts the transmission state of the central nervous system centered on the head into quantitative nervous system data based on electroencephalograms and cerebral blood flow measurement data obtained from the brain activity measurement unit 80.

A brain activity measurement unit 80 according to this embodiment is in line with the contents described in Japanese Patent No. 5283700 by the present inventor and uses the same principle.

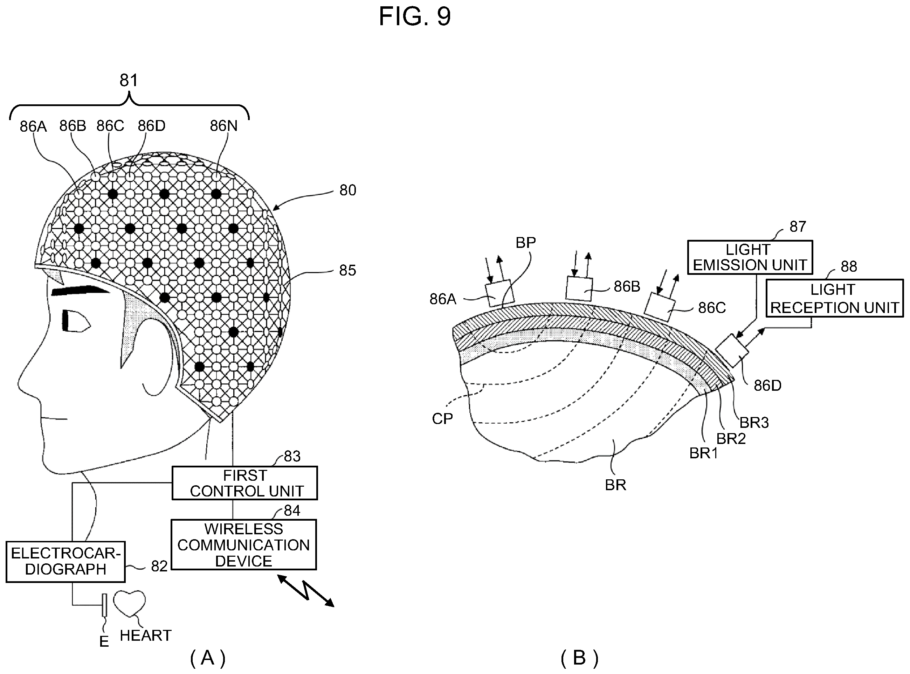

As shown in Figure 9(A), the brain activity measurement unit 80 has a blood flow measurement unit 81, an electrocardiograph 82, a first control unit 83, and a wireless communication device 84. The blood flow measurement unit 81 measures the state of blood flow by changes in the optical path and transmitted light intensity of the laser beam due to blood flow, and also measures the state of brain activity from the state of blood flow in the brain.

When laser light irradiated onto blood enters a blood layer, the laser light propagates through the blood as light including both a reflected and scattered light component due to normal red blood cells and a reflected and scattered light component due to attached thrombi. Since the amount of laser light transmitted through the blood layer varies from moment to moment depending on the state of the blood, it is possible to observe various changes in the state of the blood by continuously measuring the amount of transmitted light (amount of reflected light) and observing the changes in the amount of light.

The blood flow measurement unit 81 includes a net-like base 85 formed in a hemispherical shape according to the outline of the head to be worn on the head, and many sensor units 86A to 86N. Each of the sensor units 86A to 86N is supported at predetermined intervals by the net-like base 85 and outputs a detection signal of the amount of transmitted light measured at each measuring point on the head to the first control unit 83. The electrocardiograph 82 measures the cardiac potential generated by an electrode E affixed to the subject's skin in response to heart movement.

The first control unit 83 measures the state of brain activity (distribution of red blood cells) by deriving the displacement of blood vessels and tissue around blood vessels due to blood flow based on the light intensity when light emitted from a light emission unit 87 (Figure 9(B)) of each sensor unit 86A to 86N is received by a light reception unit 88. The first control unit 83 also stores a control program that performs arithmetic processing to cancel the component due to oxygen saturation in the signals obtained from at least two or more of the light reception units 88.

Furthermore, the first control unit 83 derives the displacement of the inner wall of the blood vessel based on the displacement of the blood vessel and the tissue surrounding the blood vessel. Furthermore, the first control unit 83 acquires the pulse wave velocity at each measurement position from the phase difference between the waveform of the electrocardiogram signal of the electrocardiograph 82 and the waveform of the detection signal obtained from the light reception unit and derives the displacement state of the inner wall of the blood vessel from the pulse wave velocity.

The wireless communication device 84 wirelessly transmits the measurement results (blood flow data) output from the first control unit 83 to the vital sign measurement apparatus 70. The brain activity measurement unit 80 has the optical sensor units 86A to 86N arranged on the net-like base 85, so that blood flow throughout the head can be measured simultaneously.

When measuring the vascular characteristics of the brain, as shown in Figure 9(B), the first control unit 83 selects any sensor unit 86D from the many arrayed sensor units 86A to 86N and emits a laser light from the light emission unit 87 of the sensor unit 86D. The laser light emitted from the light emission unit 87 is output at a wavelength λ (λ ≈ 805 nm), which is not affected by oxygen saturation.

In the brain activity measurement unit 80, when the blood flow measurement unit 81 is attached to the subject's head, the elasticity of the net-like base 85 holds the plurality of sensor units 86A to 86N in position at each measurement point on the head and with each measurement surface facing the head surface. When the blood flow measurement unit 81 is worn on the head, the plurality of sensor units 86A to 86N irradiate light onto the surface of the brain and measure the pulse wave propagation velocity in the arteries of the brain from changes in the amount of received light that propagates through the brain to measure the vascular characteristics (rate of elasticity of the blood vessel, amount of plaque in the blood vessel, rate of arterial stiffness) of the middle cerebral artery and anterior cerebral artery.

The principle of measuring pulse wave propagation velocity of cerebral arteries is explained below. For example, pulse wave propagation velocity due to blood flow through the middle cerebral artery and anterior cerebral artery is detected by each of the sensor units 86A to 86N, which are arranged corresponding to the positions to be measured that receives light propagated through the brain when light is irradiated. As a measurement method, a method is used in which a waveform of a cardiac potential obtained from the electrocardiograph 82 is compared with waveforms of signals output from the respective sensor units 86A to 86N at measurement target positions, a pulse wave propagation velocity is obtained from a phase difference, and vascular characteristics corresponding to the pulse wave propagation velocity are derived.

The principle of detecting vascular characteristics from blood flow in the brain is also explained. As shown in Figure 9(B), the brain BR is covered by spinal fluid BR1, skull BR2, and scalp BR3. Some of the laser light emitted from the light emission unit 87 of each of the sensor units 86A to 86N of the blood flow measurement unit 81 is reflected by the scalp BR3, but the remaining light penetrates the scalp BR3, skull BR2, and spinal fluid BR1 and travels inside the brain BR. The light irradiated to the head that has traveled to the brain propagates in the radial direction (depth and radial direction) in a circular arc pattern CP, as shown by the dashed lines in the figure.

Light propagation through the brain decreases in light transmittance as the light propagation path lengthens the further radially away from a base point BP where the laser light was irradiated. Therefore, the light-receiving level (amount of transmitted light) of the sensor unit 86B, which is adjacent to the sensor unit 86A on the emitting side and separated by a predetermined distance, is strongly detected. The light-receiving level (amount of transmitted light) of the sensor unit 86C, which is located next to the sensor unit 86B and separated by a predetermined distance, is detected weaker than that of the sensor unit 86B. The light reception unit 88 of the sensor unit 86A on the emitting side also receives light from the brain.

The first control unit 83 uses the detection signals corresponding to the light intensity received by the plurality of sensor units 86A to 86N as measurement data and compares their waveforms with the waveform of the cardiac potential signal from the electrocardiograph 82 to derive the vascular characteristics at each measurement position. Performing mapping processing on these detection results can also provide graphic data showing the distribution of arterial stiffness according to pulse wave propagation velocity.

Therefore, it is possible to measure changes in blood flow through the middle cerebral artery and anterior cerebral artery using the detection signal waveforms of each sensor unit 86A to 86N, and detect pulse wave propagation velocity in the brain from the measurement data of the blood flow changes.

Thus, the vital sign measurement apparatus 70 has the brain activity measurement unit 80 that is attached to the subject's head and measures EEG and cerebral blood flow in the area to be measured on the head, and the information detection unit 75 also adds, as nervous system data, the transmission state of the central nervous system centered on the head based on EEG and cerebral blood flow measurement data obtained from the brain activity measurement unit 80. In this way, the state of transmission of the central nervous system from the subject's brain can also be quantitatively acquired as data, and the subject's progress state and transition state can be monitored.

As a result, the state of transmission of the central nervous system from the subject's brain can also be quantitatively acquired as data, and the subject's progress state and transition state can be monitored.

### (3-3) Detection Method of Muscular and Skeletal System Information

The human information acquisition unit 62 has, as an additional configuration of the vital sign measurement apparatus 70, a biological signal measurement wearing device 90 worn by a subject and a joint periphery detection unit (not shown). The joint periphery detection unit includes an actuator that is driven actively or passively in association with limb movements of a subject, and detects physical quantities around joints accompanying the limb movements of the subject based on output signals from the actuator.

The physical quantities around joints include at least one or more parameter identification results among generated muscle forces produced in a musculoskeletal system, ranges of motion of respective joints, movement velocities and response velocities, autonomous control characteristics with respect to disturbances, moments of inertia of a frame, mass, and a center of gravity, impedance adjustment results of a joint system including flexor muscles and extensor muscles (viscous characteristics due to friction), electrical physical quantities (command signals), and the like.

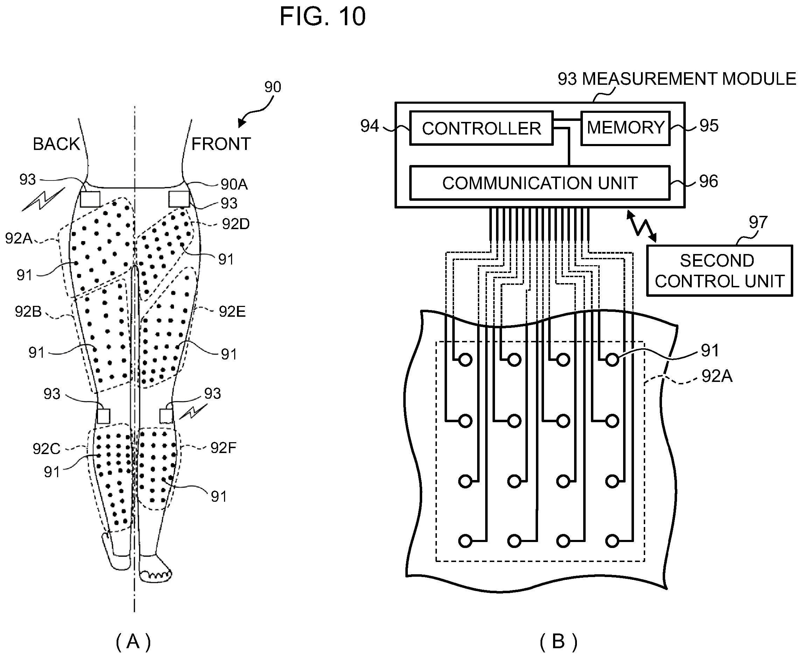

The biological signal measurement wearing device 90 worn by a subject is in line with the contents described in Japanese Patent No. 5409637 by the present inventor and uses the same principle. Figure 10(A) shows the biological signal measurement wearing device 90 according to this embodiment. The biological signal measurement wearing device 90 has a wearing device body 90A that is formed so as to cover a body surface of a subject and is worn by the subject, and on an inner surface of the wearing device body 90A (a surface that contacts the body surface of the subject in a worn state), the biological signal measurement wearing device 90 includes a biological signal sensor 91 that detects biological potential signals at least one position at which biological signals can be measured from the body of the subject.

A biological signal is a signal originating from electricity generated within a body of a subject, is a signal measurable from the body, and is a signal that changes in time series in association with bodily movements and signals from a brain. For example, biological signals mean signals generated by biological activities, such as neural potentials, myoelectric potentials, electroencephalograms, and cardiac potentials, potentials generated by motion artifacts (effects of movement), potentials generated by biochemical reactions, and vibrations such as pulse waves generated by heartbeats. The generation potential (amplitude, density, time delay) and the site of generation of the biological signal are also taken into account for quantitative data generation.

In Figure 10(A), the biological signal measurement wearing device 90 in this example has a symmetrical structure, so the left half of the figure shows the structure behind the left leg and the right half of the figure shows the structure in front of the left leg. The wearing device body 90A is provided with biological signal sensor groups 92A to 92F each including a plurality of biological signal sensors 91 that detect biological signals of a subject. The biological signal sensors 91 are arranged at equal intervals along the muscle flow of the subject's legs.

The plurality of biological signal sensors 91 are arranged on the wearing device body 90A along a flow of a gluteus maximus muscle at a portion corresponding to buttocks of a subject (biological signal sensor group 92A). Similarly, at a portion corresponding to a rear side of a thigh of the subject (biological signal sensor group 92B), biological signal sensors are arranged along flows of a biceps femoris muscle, a semimembranosus muscle, and a semitendinosus muscle, and at a portion corresponding to a calf (biological signal sensor group 92C), biological signal sensors are arranged along a flow of a triceps surae muscle. Also, in a region corresponding to the anterior side of the hip joint (biological signal sensor group 92D), sensors are arranged along the course of the adductor longus and the iliopsoas; in a region corresponding to the anterior side of the thigh (biological signal sensor group 92E), sensors are arranged along the course of the quadriceps femoris; and in a region corresponding to the shin (biological signal sensor group 92F), sensors are arranged along the course of the tibialis anterior, the soleus, and the extensor digitorum longus.

Here, Figure 10(B) is a diagram showing an example of a measurement module connected to biological signal sensor groups in the wearing device body 90A. Since all biological signal sensor groups 92A to 92F in the wearing device body 90A are connected to the measurement module 93 in the same way, the biological signal sensor group 92A is used as an example here.

Figure 10(B) is a diagram schematically showing the biological signal sensor group 92A in the wearing device body 90A and the measurement module 93 connected to this biological signal sensor group 92A. Each of the biological signal sensors 91 included in the biological signal sensor group 92A is insulated from each other, and each is connected to the measurement module 93 via conductive wiring. Each of these biological signal sensors 91 is assigned an address.

The measurement module 93 includes a measurement module controller 94 to which a plurality of biological signal sensors 91 constituting the biological signal sensor group 92A are connected, the measurement module controller 56 selecting at least two biological signal sensors 91 from these biological signal sensors 91 and acquiring a biological signal by taking a difference between detection signals detected by the selected biological signal sensors 91; a memory 95 that records the acquired biological signal; and a communication unit 96 that transmits to the outside biological signals sequentially acquired and/or biological signals recorded in the memory 95. The measurement module 93 is provided for each biological signal sensor group 92A to 92F, to which the respective biological signal sensors 91 are connected.

The measurement module controller 94 has electronic circuit capable of sequentially selecting at least two biological signal sensors 91 from the plurality of connected biological signal sensors 91 in response to a command signal input via the communication unit 96 and acquiring the biological signals between these two selected biological signal sensors 91.

The measurement module controller 94 further has signal processing means such as a filter that removes or extracts predetermined frequency components from the biological signals thus acquired and an amplifier that amplifies the acquired biological signals. The biological signals thus acquired are output from the measurement module controller 94 to the communication unit 96 and/or the memory 95. When selecting the biological signal sensors 91, the measurement module controller 94 may operate each biological signal sensor 91 sequentially in a preset order, or it may select the biological signal sensor 91 at the address designated by the designation signal based on a designation signal input via the communication unit 96.

The communication unit 96 has a thin antenna and a communication circuit connected to this antenna. The communication unit 96 transmits, via an antenna, measurement information to a second control unit 97, the measurement information including a signal that includes a biological signal output from the measurement module controller 94 and information such as an address indicating position information of a biological signal sensor 91 that detected the biological signal, and/or a signal that includes a biological signal read from the memory 95 and information such as an address indicating position information of a biological signal sensor 91 that detected the biological signal.

This second control unit 97 generates a designation signal to specify which of the biological signal sensors 91 is to be selected from each of the biological signal sensor groups 92A to 92F provided in the wearing device body 90A, and signals to start and end data acquisition, etc., and selects the designated biological signal sensor 91 according to the signal and measures the biological signals.

Thus, with the biological signal measurement wearing device 90 of the present invention, the biological signal sensor groups 92A to 92F having the plurality of biological signal sensors 91 are provided in the wearing device body 90A, so that the plurality of biological signal sensors 91 can be placed at a given site at once by simply wearing the wearing device body 90A, allowing to detect biological signals at each point by placing them in close contact with the skin surface. Thus, even when biological signals are monitored at a large number of points on the body surface of a subject, it is possible to eliminate the labor of attaching and detaching the biological signal sensors 91 one by one, and to easily measure biological signals.

By detecting biological signals with the plurality of biological signal sensors 91 in this manner, biological signals can be measured at a plurality of respective points in the area where the biological signal sensor groups 92A to 92F are located. The measured data at each point can then be mapped according to the address assigned to each of the biological signal sensors 91 to measure the distribution of biological signals in the subject's body.

The second control unit 97 obtains from the biological signals acquired by the measurement module 93 the myopotential signals associated with the subject's muscle activity and the neurotransmission signals for operating the subject's musculoskeletal system.

The information detection unit 75 (Figure 4) converts, based on the physical quantities around the joint described above and one or both of the myopotential signals and neurotransmission signals obtained from the second control unit 97, the activity state of the musculoskeletal system centered on the joint and/or the transmission state of the peripheral nervous system into quantitative musculoskeletal data and/or nervous system data, and also converts the transmission state of either or both of the central nervous system and the peripheral nervous system centered on a desired site into quantitative nervous system data.

As a result, the subject can perform limb movements to obtain quantitative data on the activity state of the musculoskeletal system and the transmission state of the peripheral nervous system based on physical quantities around joints and biological signals obtained from body surface sites with reference to joints, and to monitor the subject's progress state and transition state.

In addition, biological signals are acquired from body surface sites other than sites referenced to the subject's joints, and quantitative data are acquired not only on the peripheral nervous system around the joints but also on the transmission state of the central nervous system and/or the peripheral nervous system from desired sites, thereby making it possible to monitor the progress state and transition state of the subject.

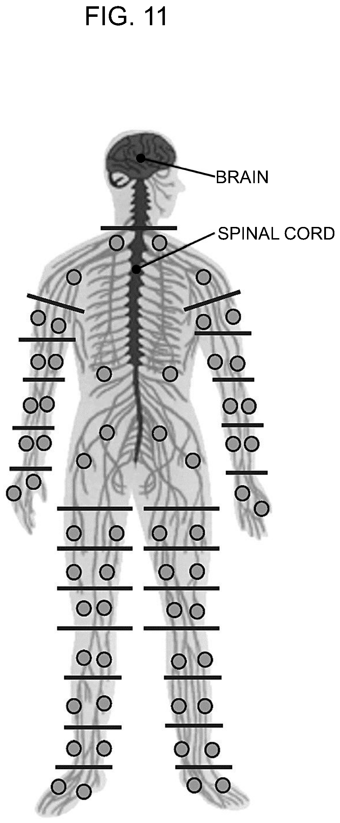

It should be noted that, for example, as shown in Figure 11, by arranging the biological signal sensor groups 92A to 92F over the entire body of the subject, it becomes possible to visualize state transitions of information transmission pathways in the cranial nervous system. In Figure 11, if the entire body of the subject is divided into units of predetermined regions (for example, regions innervated by sensory nerves such as dermatomes), neural connections throughout the entire body can be grasped from a bird's-eye view and on a region-by-region basis. Therefore, if it is possible to detect at which site a problem in nerve transmission has occurred, it becomes possible to ascertain not only the problematic site but also the degree of influence on either the central side or the peripheral side.

### (3-4)Method of Detecting Activity State of Physiological System

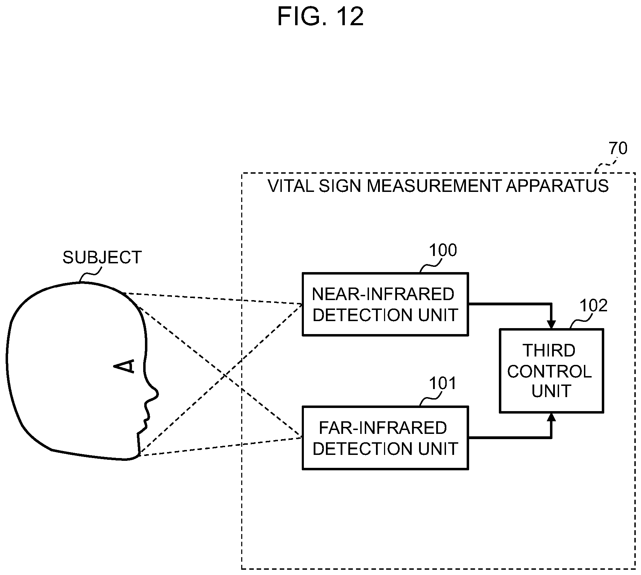

In the human information acquisition unit 62, as an additional configuration of the vital sign measurement apparatus 70, as shown in Figure 12, a near-infrared detection unit 100 and a far-infrared detection unit 101 are provided, and activity states of the physiological system and the nervous system of the subject are detected using a non-contact method.

That is, the human information acquisition unit 62 simultaneously and noninvasively measures fluctuations in the pulse rate and changes in skin temperature of the subject in daily life by irradiating infrared rays centered on the face of the subject using the near-infrared detection unit 100 and the far-infrared detection unit 101.

The near-infrared detection unit 100 irradiates near-infrared light centered on a region of interest including a cheek area of the subject's face, receives reflected light from the face, and generates a near-infrared image. In addition, the far-infrared detection unit 101 irradiates far-infrared light centered on a region of interest of the subject's face and detects the skin temperature of the face.

The third control unit 102 estimates the pulse rate of the subject based on a period of change of an intensity waveform in a region of interest among reflected light received by the near-infrared detection unit 100. The third control unit 102 extracts, from a near-infrared image generated by the near-infrared detection unit 100, regions of the subject's face that are susceptible to activity of the autonomic nervous system and regions that are less susceptible thereto, respectively, and calculates a temperature difference in skin temperature for each region based on detection results of the far-infrared detection unit 101.

### A. Pulse Measurement Method Based on Infrared Light

In biological tissues, light-absorbing substances are water and hemoglobin in blood. Water has a strong absorption property in infrared rays with wavelengths longer than 1350 nm, while hemoglobin has a strong absorption property in visible light with wavelengths shorter than 650 nm. In noninvasive biological diagnosis using light, light in an infrared to near-infrared region having wavelengths from 650 nm to 1350 nm, which have high biological permeability, is irradiated, and reflected light or transmitted light containing biological information is often measured.

A pulse is a physiological phenomenon in which a volume of a blood vessel changes due to blood pressure work caused by cardiac output. A change in a volume of a blood vessel is caused by a change in blood flow volume. Because a change in blood flow volume causes a change in an amount of light absorbed by hemoglobin in blood, it is possible to estimate a pulse rate from a change period of transmitted light or reflected light of light irradiated onto a living body.

In the present invention, without requiring the subject to wear a measurement device and in order to perform measurements regardless of day or night, a change period of reflectance of near-infrared light irradiated onto a living body is measured by a near-infrared camera, and a pulse rate is estimated. Since near-infrared light is invisible, measurement can be performed even at night without imposing a burden on the subject to be measured. In order to estimate a stress state from a pulse rate, it is necessary to measure a pulse rate that finely fluctuates due to activity of the autonomic nervous system.

The third control unit 102 implements an algorithm that extracts a waveform period required for calculating a pulse rate from waveform data obtained from the near-infrared detection unit 100.

The near-infrared detection unit 100 measures the intensity of the reflected light reflected by the irradiated near-infrared rays in the living tissue. The near-infrared detection unit 100 performs measurement for a subject at rest, while minute body movements and facial movements due to respiration are superimposed on a pulse waveform as motion artifacts representing changes in an amount of reflected light.

To remove these motion artifacts, a digital filter is first applied to the pulse rate frequency band. A range of a pulse rate at rest is set to 35 to 180 bpm, and motion artifacts may be superimposed on a measured waveform also in a band of 0.5 to 3.0 Hz, while an amplitude of a waveform superimposed on a pulse waveform is significantly large.

Therefore, when a variance value of waveform data within a certain period exceeds a threshold, the third control unit 102 recognizes that the subject is in motion and removes data during that period as data having low reliability. A waveform after band-pass filtering has a bimodal characteristic due to arterial blood flow volume. In order to facilitate feature point extraction, a moving average filter is applied to convert a two-peak waveform into a single-peak waveform.

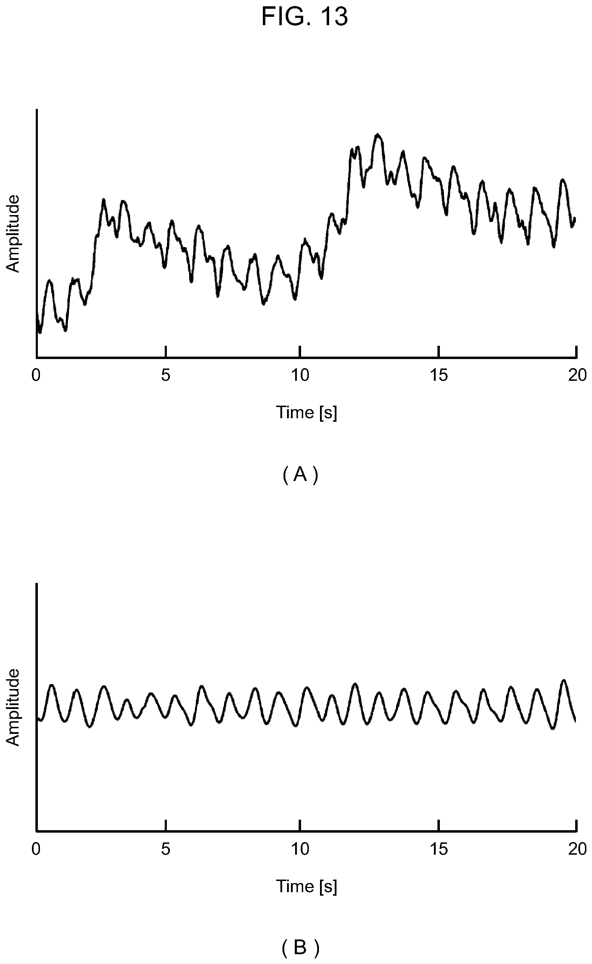

Waveforms before and after application of a band-pass filter and a moving average filter to a measured waveform are shown in Figure 13(A) and Figure 13(B), respectively. It can be seen that motion artifacts superimposed on the pulse waveform are removed by filtering.

Next, as methods for calculating a pulse rate from an intensity waveform of light measured by a near-infrared camera, two major methods are generally known.

In a first method, a portion of a face is set as a region of interest (ROI), and, among frequency spectra obtained by a spectral analyzer from intensity data of reflected light of the cheek portion for 30 seconds acquired by measurement using an RGB camera, a frequency considered to be a pulse wave component is compared with a frequency derived from a pulse rate indicated by a pulse oximeter. However, noise caused by body motion is significantly large relative to a pulse waveform. When attempting to calculate a pulse rate by such frequency analysis, it is necessary to greatly restrict behavior of a subject to be measured.

On the other hand, in a second method, a time required for one pulse beat is calculated from a period of feature points of a waveform detected from time-series data of a measured waveform, and a pulse rate is calculated by dividing 60 by that time. Compared with a method of calculating a pulse rate from frequency analysis that requires continuous measurement data for a certain period of time, a method of calculating a pulse rate from a period of measured feature points enables removal of motion artifacts by removing a feature point period erroneously detected due to noise caused by body motion. In addition, since a minimum measurement time required is shorter than that for frequency analysis and a pulse rate can be calculated from several beats, it is possible to calculate a pulse rate at shorter time intervals.

However, since a pulse rate at rest of a healthy person is 60 to 80 bpm, a period of feature points obtained by measurement for 5 seconds is 5 to 7 beats, and there is a problem in that a number of obtained data points are small in order to calculate a more accurate pulse rate. In addition, as shown in Figure 12(A), since a waveform of intensity variation is bimodal in synchronization with arterial blood flow volume, erroneous detection of a period of feature points is caused, which becomes a factor that causes an error in a calculated pulse rate.

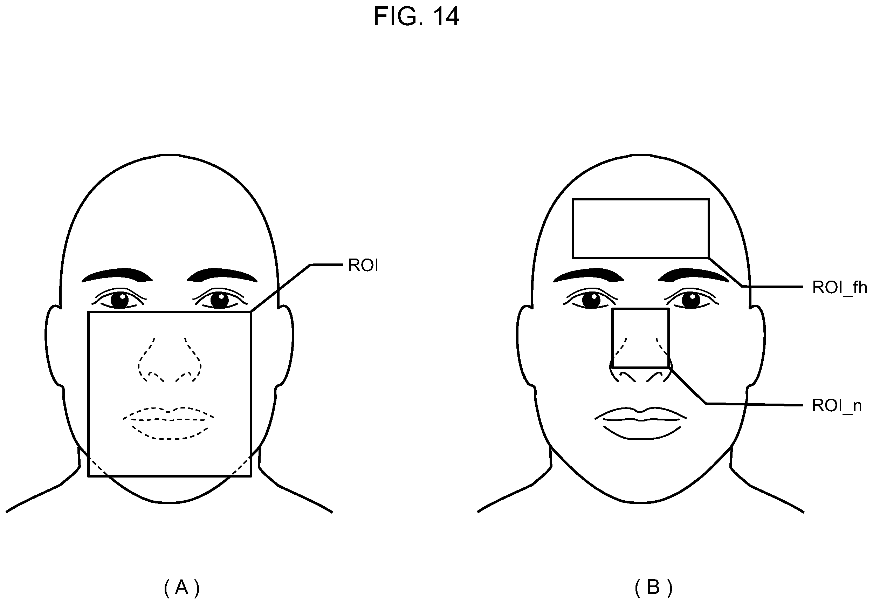

For daily measurement, a biological site to be measured needs to be exposed regardless of day or night. Therefore, in the vital sign measurement apparatus 70 according to the present invention, ROI is set to a cheek portion of a person. The location of the set ROI is shown in Figure 14(A). An average of intensity values measured at pixels within the set ROI is used as a measured value, and a temporal change of the measured value is recorded as pulse waveform data. As periods of feature points of waveform data, not only an upper-end interval tₜₚ of a waveform but also a lower-end interval t_{bp} is added as another feature point, thereby increasing feature points serving as reference data for calculating a pulse rate.

Next, in order to extract a feature point period required for calculating a pulse rate, an algorithm for removing erroneously detected tₜₚ and t_{bp} that do not indicate a pulse period from among detected tₜₚ and t_{bp} is described below in (a) to (f). Here, a set before removal is defined as S₀.
(a) For each of tₜₚ and t_{bp}, when a peak interval tᵢ varies by 30% or more relative to an immediately preceding peak interval tᵢ₋₁, the peak interval tᵢ is removed from set S₀. A set S₁ of selected peak intervals is represented by Equation (1) shown below.
   [Equation 1] ${S}_{1} = \left\{\left.{t}_{i}\right|\left|{t}_{i}-{t}_{i - 1}\right|<0.3{t}_{i - 1}, {t}_{i}∈{S}_{0}\right\}$ (1)
(b) A standard deviation σ₀ of set S₀ is calculated, and peak intervals tᵢ that vary within a range of 2σ₀ are removed from set S₁. A set S₂ of selected peak intervals is represented by Equation (2) shown below.
   [Equation 2] ${S}_{2} = \left\{\left.{t}_{i}\right|\left|{t}_{i}-{t}^{˜}\right|<2{σ}_{0},{t}_{i}ϵ{S}_{1}\right\}$ (2)
(c) A standard deviation σ₂ of set S₂ is calculated, and peak intervals tᵢ that vary within a range of σ₂ are removed from set S₂. A set S₃ of selected peak intervals is represented by Equation (3) shown below.
   [Equation 3] ${S}_{3} = \left\{\left.{t}_{i}\right|\left|{t}_{i}-{t}^{˜}\right|<{σ}_{2},{t}_{i}ϵ{S}_{2}\right\}$ (3)
(d) A standard deviation σ₃ of set S₃ is calculated, and peak intervals tᵢ that vary within a range of σ₃ are removed from S₃. A set S₄ of selected peak intervals is represented by Equation (4) shown below.
   [Equation 4] ${S}_{4} = \left\{\left.{t}_{i}\right|\left|{t}_{i}-{t}^{˜}\right|<{σ}_{3},{t}_{i}ϵ{S}_{3}\right\}$ (4)
(e) Peak intervals blood remaining in set S₄ are converted into pulse rates, and a histogram with bins of 5 bpm is generated. Here, while a width of a pulse rate of one bin of the histogram is uniform, a width of a peak interval corresponding to the pulse rate differs. Accordingly, a weighted histogram is generated using Equation (5) shown below.
   [Equation 5] $h {′}_{i - i + 5} = \frac{{t}_{i - i + 5}}{{t}_{30 - 35}} {h}_{i - i + 5}$ (5)
   Here, t₃₀₋₃₅ represents a time width of a peak interval for a pulse rate from 30 bpm to 35 bpm, tᵢ₋ᵢ₊₅ represents a time width of a peak interval for a pulse rate from i bpm to i+5 bpm, hᵢ₋ᵢ₊₅ represents a frequency of peak intervals before correction for a pulse rate from i bpm to i+5 bpm, and h'ᵢ₋ᵢ₊₅ represents a corrected value of a frequency of peak intervals for a pulse rate from i bpm to i+5 bpm.
(f) With respect to values of the weighted histogram, a window having a width of 20 bpm is set so that a sum of frequencies of four adjacent bins is maximized, and peak intervals tᵢ outside the window are removed.

According to the above-described algorithm, an average value of extracted peak intervals is set as a peak interval t within a measurement period, and a value obtained by dividing 60 seconds by the peak interval t is set as a pulse rate.

In this manner, the third control unit 102 sets feature points at an upper end and a lower end, respectively, of an intensity waveform of reflected light obtained from the near-infrared detection unit 100, and estimates a pulse rate based on peak intervals between upper ends and between lower ends, which are periods of the respective feature points. In addition, the third control unit 102 calculates a standard deviation of a plurality of peak intervals and removes peak intervals that do not indicate a pulse period based on the standard deviation. Furthermore, the third control unit 102 generates a weighted histogram in predetermined time units from pulse rates converted based on remaining peak intervals after removal, and corrects a frequency of the peak intervals based on the histogram.

Thus, the third control unit 102 can calculate an accurate pulse rate from a measured waveform of pulse waveform data for a relatively short period of time based on a group of pixels within the set ROI.

### B. Skin Temperature Measurement Using a Far-Infrared Detection Unit

Heat generated inside a living body is transported to a body surface by conduction and convection. However, since heat conduction by biological tissue itself is poor and acts as thermal insulation, most transport of heat to the skin is considered to be due to skin blood flow. Skin blood flow volume varies due to activity of the autonomic nervous system, mainly through vasoconstriction and vasodilation effects of the sympathetic nervous system and the parasympathetic nervous system.

In conventional prior studies, attempts have been made to estimate activity of the autonomic nervous system by measuring changes in skin temperature. However, since a facial heat distribution is greatly affected by a hairstyle of the subject to be measured and whether the subject to be measured wears glasses, the human information acquisition unit according to the present invention is configured to set an appropriate measurement site for estimating activity of the autonomic nervous system based on a near-infrared image.

In order to estimate a stress state from changes in skin temperature, it is necessary to set, as ROI to be measured, a facial site at which temperature changes due to stress prominently appear. Among facial sites, arteriovenous anastomoses (AVA), which are highly susceptible to activity of the autonomic nervous system, are particularly concentrated in a nasal region, making the nasal region suitable for skin temperature measurement for the purpose of estimating a stress state.

In addition, since skin temperature is affected by ambient air temperature, changes in skin temperature need to be recorded as relative temperature. At this time, ROI measured as a reference for changes in skin temperature of the nasal region needs to be set at a site that is less susceptible to activity of the autonomic nervous system. As a facial site having a low density of AVA and being less susceptible to activity of the autonomic nervous system, a forehead region can be cited.

Based on the above, in the third control unit 102 according to the present invention, changes in skin temperature are recorded as a temperature difference between a region of interest ROI_n of a nasal region and a region of interest ROI_fh of a forehead region. Locations of the respective ROIs are shown in Figure 14(B). In addition, an equation for calculating a temperature change is shown in Equation (6).
[Equation 6] ${T}_{r} = {T}_{n} - {T}_{fh}$ (6)

Here, Tₙ represents an average value of skin temperatures measured at all pixels within ROI_n, T_{fh} represents an average value of skin temperatures measured at all pixels within ROI_fh, and Tᵣ represents a relative temperature between the forehead region and the nasal region. In setting each ROI, coordinates of a nasal region and a forehead region in a near-infrared image are converted into coordinates in a far-infrared image.

Specifically, in performing coordinate conversion from a near-infrared (NIR) image to a far-infrared (FIR) image, since a near-infrared camera and a far-infrared camera differ in resolution and angle of view, coordinate information of the near-infrared image is associated with pixels of the far-infrared image.

That is, an x-coordinate X_{fir} on a far-infrared image is expressed as shown in the following Equation (7), where a horizontal angle of view of a near-infrared camera is θ_{nir_h} and a horizontal angle of view of a far-infrared camera is θ_{fir_h}.
[Equation 7] ${x}_{fir} = \frac{40}{tan \frac{{θ}_{fir_h}}{2}} \left(\frac{tan \frac{{θ}_{nir_h}}{2}}{320}{x}_{nir}+tan\frac{{θ}_{fir_h}}{2}-tan\frac{{θ}_{nir_h}}{2}\right)$ (7)

In addition, a y-coordinate Yfir on a far-infrared image is expressed as shown in the following Equation (8), where a vertical angle of view of a near-infrared camera is θ_{nir_v} and a vertical angle of view of a far-infrared camera is θ_{fir_v}. Here, d represents a difference between optical axes, and L represents a distance to an object.
[Equation 8] ${y}_{fir} = \frac{30}{tan \frac{{θ}_{fir_v}}{2}} \left(\frac{tan \frac{{θ}_{nir_v}}{2}}{240}{y}_{nir}+tan\frac{{θ}_{fir_v}}{2}-tan\frac{{θ}_{nir_v}}{2}-\frac{d}{L}\right)$ (8)

Thus, following automatic detection results of a measurement target site using image information of the near-infrared detection unit 100, the third control unit 102 can accurately calculate a relative temperature difference between skin temperatures of a forehead region and a nasal region of a subject based on image information of the far-infrared detection unit 101.

### C. Respiration Measurement Based on Skin Temperature

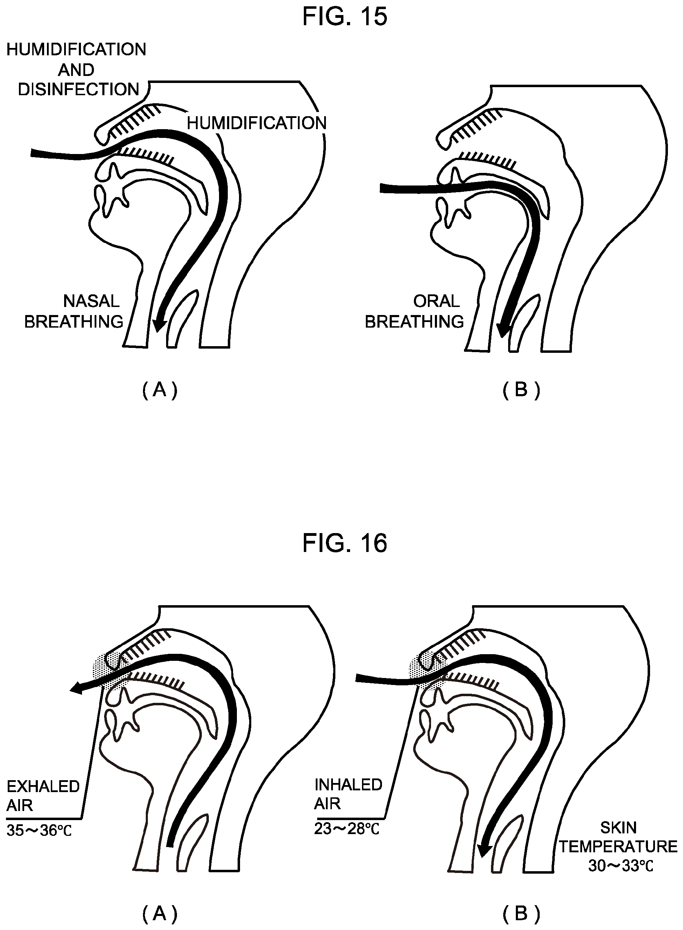

Respiration is related to functions of the autonomic nervous system and immunity, and is used for diagnosis of sleep disorders. Respiration includes nasal breathing (Figure 15(A)) and oral breathing (Figure 15(B)), and nasal breathing is originally preferable. In the case of oral breathing, inhaled air does not pass through cilia and mucosa of the nasal cavity. In addition, oral breathing causes various diseases, including sleep apnea syndrome (SAS).

As shown in Figure 16(A) and Figure 16(B), nostrils and an oral cavity are warmed by exhaled air (35 to 36 °C) and cooled by inhaled air (23 to 28 °C). Therefore, a region of interest (ROI) is set at nostrils and an oral cavity of a subject, and temperature changes of the nostrils and the oral cavity due to respiration are measured by a far-infrared detection unit (a far-infrared camera). Since a surface temperature of skin is affected by ambient air temperature, measurement is performed as a relative temperature between nostrils and an oral cavity.

In practice, since automatic detection of a measurement target site based on a skin temperature distribution map is difficult, as described above, automatic detection of a measurement target site (nostrils and an oral cavity) is performed using image information of a near-infrared detection unit (camera), and a region of interest (ROI) is set. Then, while a nasal temperature in nasal breathing is relatively high, an oral temperature in oral breathing is relatively low.

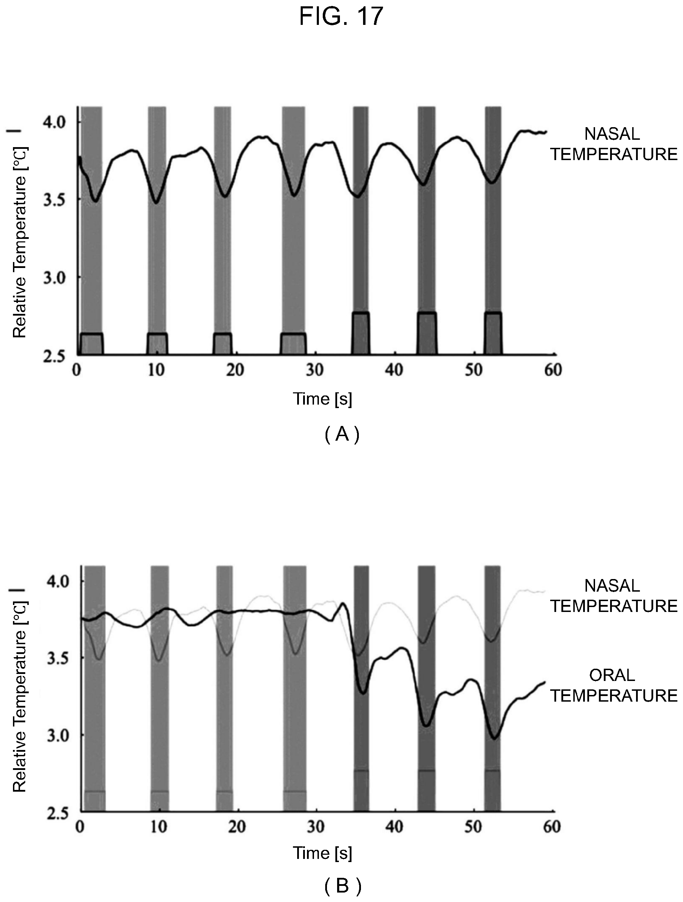

In practice, after nasal breathing and oral breathing by a subject were consecutively measured for 30 seconds each, a one-minute break was provided, and the same respiration measurement was repeated four times. As a result, as shown in Figure 17(A), a decrease in nasal temperature due to inhalation in nasal breathing was observed. In addition, as shown in Figure 17(B), a decrease in oral temperature corresponding to oral breathing was observed.

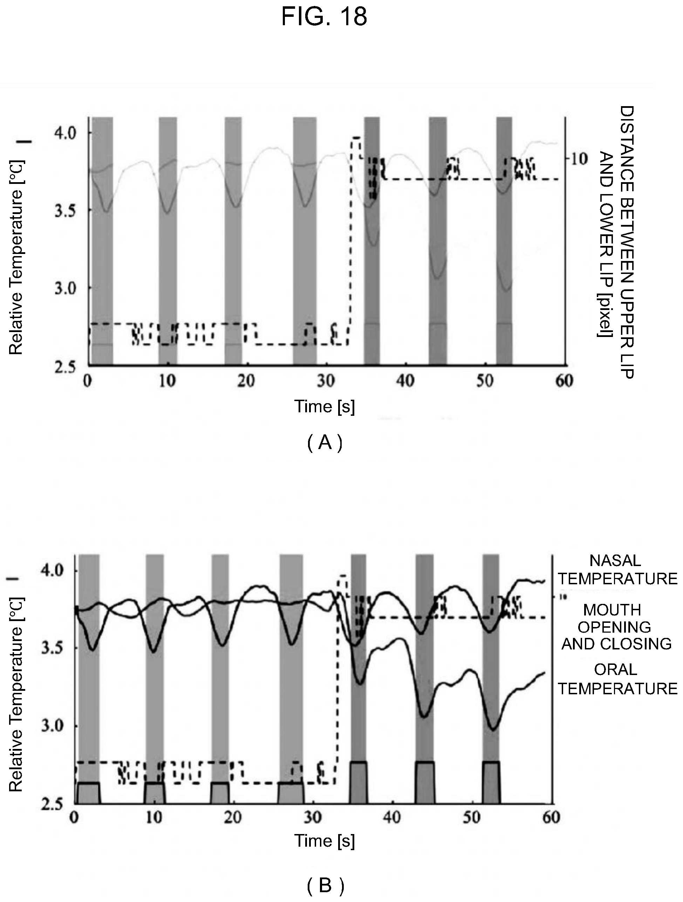

Furthermore, as shown in Figure 18(A), opening and closing of the mouth due to a change in a breathing method from nasal breathing to oral breathing was observed based on a distance between an upper lip and a lower lip. When the graphs of Figure 17(A), Figure 17(B), and Figure 17(A) are superimposed and displayed, they are represented as shown in Figure 18(B).

It should be noted that, as a method for extracting an inhalation timing, first, high-frequency noise is removed from measurement results of a relative temperature between nostrils and an oral cavity using a moving average filter, and then first-order differentiation is performed to extract a temperature decrease timing due to exhalation.

In addition, when a nasal temperature and an oral temperature decrease at the same timing, since air inflow through a nasal cavity pathway occurs due to alveolar pressure becoming negative relative to atmospheric pressure, it becomes possible to determine oral breathing.

In this manner, it is possible to realize calculation of a respiration rate and determination of a respiration method by a non-contact measurement method. Although simultaneous measurement of nasal breathing and oral breathing is possible, by executing an algorithm that takes characteristics of temperature changes during each breathing into account, it is possible to realize accurate calculation of a respiration rate and determination of a respiration method.

### D. Oxygen Saturation Using Two Types of Near-Infrared Wavelengths

Conventionally, a pulse oximeter is known as a measurement device that utilizes light absorption characteristics of hemoglobin in blood. A pulse oximeter is a medical device that is attached to a fingertip to measure blood oxygen saturation and a pulse rate.

In the present invention, since blood oxygen saturation has a property of varying depending on a respiration rate, a degree of binding between hemoglobin and oxygen, and cardiac output, attention is focused on a difference in absorption characteristics between oxygenated hemoglobin (HbO₂) and reduced hemoglobin (Hb).

In a conventional measurement method, infrared light (with a wavelength around 660 nm) and near-infrared light (with a wavelength around 900 nm) are irradiated onto a fingertip from a light-emitting element, and blood oxygen saturation is estimated from a ratio of amounts of transmitted light measured by a light-receiving element, while a pulse rate is also estimated using periodic changes in an amount of the transmitted light. Infrared light, which is visible light, has been used because a difference in absorptivity between oxygenated hemoglobin (HbO₂) and reduced hemoglobin (Hb) is large.

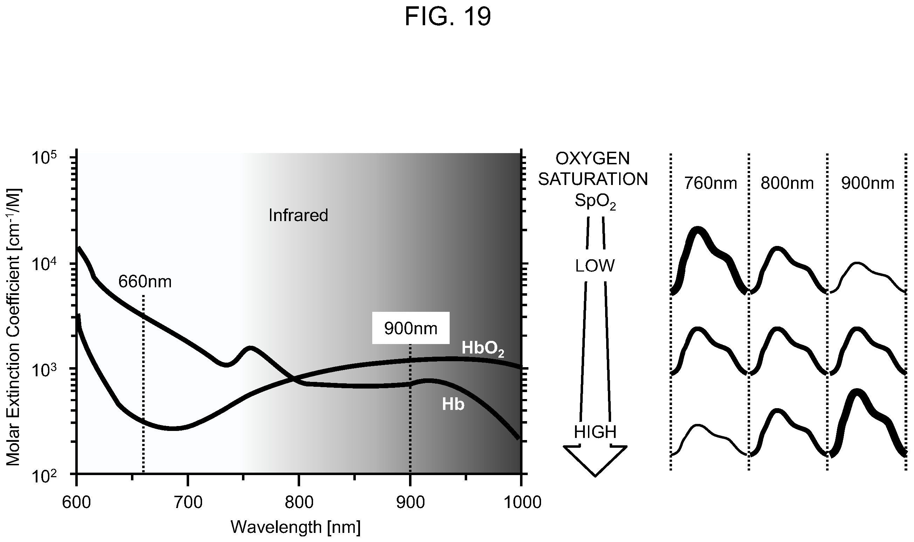

However, in the present invention, non-contact measurement is performed using only near-infrared (NIR) light, which is invisible light (Figure 19). As a result, by alternately blinking light-emitting elements that irradiate two types of near-infrared light having wavelengths of 800 nm (or 760 nm) and 900 nm, and imaging irradiation sites (ROI) of the respective light-emitting elements with a single near-infrared camera (near-infrared detection unit 100), it is possible to measure blood oxygen saturation centered on ROI of a subject in a non-contact manner even in a dark environment.

### (3-5) Method of Detecting an Activity State of a Nervous System

The autonomic nervous system includes a sympathetic nervous system that functions when a living body is in a tense state or an active state, and a parasympathetic nervous system that functions when the living body is in a resting state. While a blood pressure value and a pulse rate increase in a state in which the sympathetic nervous system is dominant, a blood pressure value and a pulse rate decrease in a state in which the parasympathetic nervous system is dominant; therefore, autonomic nervous function and cardiac function have a high correlation.

As a method for measuring autonomic nervous function, there is a method for measuring sympathetic nervous function of the heart using heart rate variability; specifically, a CVRR (Coefficient of Variation of R-R intervals) method in which a coefficient of variation is obtained and evaluated using R-R intervals of an electrocardiogram waveform, and, secondly, a method in which a power ratio of frequency components (a high-frequency component and a low-frequency component) of heart rate variability is used as an index of sympathetic nervous activity.

In addition, there is also a method for measuring autonomic nervous function by measuring sympathetic nervous function of a vascular system using a pulse wave. As this measurement method, there is a method in which a magnitude of amplitude fluctuation of a photoplethysmographic (PPG) waveform is calculated as an evaluation value of autonomic nervous function.

In the present invention, the third control unit 102 can contribute to evaluation of autonomic nervous function of a subject (such as presence or absence of imbalance) based on all or a combination of a pulse rate of the subject detected using the near-infrared detection unit 100 and the far-infrared detection unit 101 constituting the human information acquisition unit 62, a relative temperature difference between skin temperatures of a forehead region and a nasal region of the subject, a respiration rate and a respiration method of the subject, and an oxygen saturation of the subject.

### (3-6) Method of Detecting Psychological Information

In the human information acquisition unit 62, the information detection unit 75 analyzes facial expressions and voice of a subject using a facial imaging camera 71 and a sound collection microphone 72 to detect psychological information.

Specifically, the information detection unit 75 detects facial expressions of a subject by executing a facial analysis algorithm, including not only expressions such as sadness, joy, anger, empathy, and frivolity, but also flushed skin, goosebumps, a frequency of eyelid opening and closing, tears, and the like, and simultaneously detects a tone of voice and a pitch of voice (such as a voice tone and a sense of excitement) of the subject by executing a voice analysis algorithm.

This facial analysis algorithm is designed to approximate human interpretation (experience, preconception, and prediction), and is an algorithm obtained from accumulated results regarding how emotions and psychology are reflected in facial expressions based on states and changes of a human face.

Similarly, the voice analysis algorithm is designed to approximate human interpretation (experience, preconception, and prediction), and is an algorithm obtained from accumulated results regarding how emotions and psychology are reflected in voice based on a tone and a pitch of a human voice.

By using this voice analysis algorithm, it becomes possible to estimate a change in a psychological state of a subject through daily communication of the subject and analyses thereof and narrative analysis, based on keywords used in utterances of the subject, intonation during utterances, loudness of voice, a state of narrative structure, continuity or discontinuity of communication, and differences from accumulated data thereof.

In addition, the information detection unit 75 can also estimate a psychological state of a subject based on personal information of the subject (such as heart rate, electrocardiogram, pulse rate, blood pressure, body temperature, skin blood flow distribution, skin temperature distribution, perspiration, and odor substances), which are detection results obtained by the above-described vital sign measurement apparatus 70, and environment information of the subject, which are detection results obtained by a peripheral measurement device 74 described below.

In this manner, the information detection unit 75 can comprehensively estimate and grasp psychological information of a person based on both personal information obtained by sensing and personal information obtained by communication analysis and narrative analysis.

### (3-7) Method of Detecting an Activity State of a Motor System (Action Information and Behavior Information)

In the human information acquisition unit 62 (Figure 4), the information detection unit 75 detects motion data recognized by a motion capture device 73 as an activity state of a motor system of a subject.

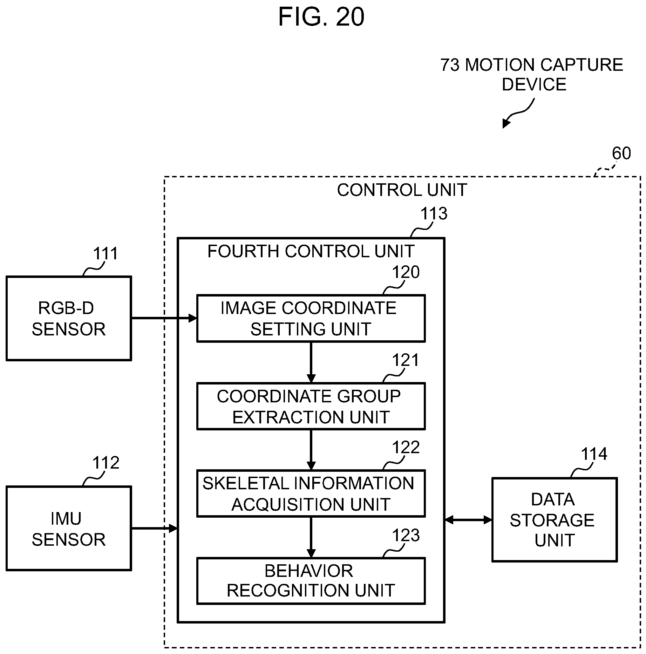

Here, as shown in Figure 20, the motion capture device 73 is arranged in indoor facilities such as a home of a subject, and includes an RGB-D sensor 111 and an IMU (Inertial Measurement Unit) sensor 112 connected to the control unit 60 described above. The IMU sensor 112 includes a three-axis acceleration sensor, a three-axis angular velocity sensor, and a geomagnetic sensor, and is capable of measuring three-axis acceleration, angular velocity, and geomagnetism.

The RGB-D sensor 111 has a depth sensor capable of measuring a distance from the camera to a target object in addition to an RGB color camera function, and is capable of performing three-dimensional scanning of the target object. As the RGB-D sensor 111, for example, when a LiDAR camera L515 of RealSense (a trademark of Microsoft Corporation of the United States) is applied, the depth sensor includes a LiDAR sensor, and measures a time until laser light irradiated is reflected back after hitting an object to measure a distance and a direction to the object.

In practice, the RGB-D sensor 111 is arranged to capture an entire body of the subject with a standing position of the subject in indoor facilities as a reference, and sequentially acquires an RGB image and a depth image as imaging results.

The control unit 60 includes a fourth control unit 113 under control of the overall control unit 50 described above, and a data storage unit 114 in which various data are database-managed so as to be readable and writable in accordance with instructions from the fourth control unit 113.

The fourth control unit 113 includes an image coordinate setting unit 120, a coordinate group extraction unit 121, a skeletal information acquisition unit 122, and a behavior recognition unit 123. The image coordinate setting unit 120 sets, as image coordinates, a plurality of skeletal representative points assigned to an entire body of a subject while estimating a posture of the entire body of the subject based on RGB images sequentially acquired from the RGB-D sensor 111.

Specifically, the image coordinate setting unit 120 sets an image coordinate group in a two-dimensional coordinate system in which one of left and right shoulder portions of the subject is defined as a coordinate origin, a direction of a line connecting the left and right shoulder portions is defined as an X-axis with respect to the coordinate origin, and a vertical direction with respect to the X-axis is defined as a Z-axis. That is, the coordinate origin is moved to a right shoulder portion or a left shoulder portion, and an XZ plane is rotated so as to coincide with a line connecting the left and right shoulder portions. As a result, it becomes possible to perform action recognition with improved robustness regardless of a position of the RGB-D sensor 111.

The coordinate group extraction unit 121 sequentially extracts, from among image coordinate groups set by the image coordinate setting unit 120, image coordinate groups related to behavior of the subject based on a transition state of posture of the subject. In practice, the coordinate group extraction unit 121 needs to extract key points of an entire body as extraction targets from behaviors, assuming that clothing of the subject and a surrounding environment may change.

Accordingly, the coordinate group extraction unit 121 adopts a lightweight OpenPose architecture using a trained model (a system that estimates a human skeleton by deep learning) to obtain whole-body posture estimation and image coordinates of skeletal representative points of both hands and fingers. For example, for an upper body of a subject, the coordinate group extraction unit 121 sequentially extracts, as an image coordinate group, skeletal representative points totaling 60 points, including 18 points of a torso of the upper body of the subject and 42 points of both hands and fingers (21 points per hand). It should be noted that, by using only stages 1 and 2 among six stages of the OpenPose architecture, it is possible to reduce a computational load by 32.4%.

The skeletal information acquisition unit 122 acquires temporally continuous three-dimensional skeletal information centered on both hands and fingers of a subject by time-synchronizing image coordinate groups sequentially extracted by the coordinate group extraction unit 121 with depth images sequentially acquired from the RGB-D sensor 111.

Specifically, the skeletal information acquisition unit 122 acquires three-dimensional skeletal information by configuring a three-dimensional coordinate system in which a depth direction in depth images sequentially acquired from the RGB-D sensor 111 is defined as a Y-axis, together with a two-dimensional coordinate system of image coordinate groups sequentially extracted by the coordinate group extraction unit 121.

A conversion formula from an image coordinate system of a two-dimensional coordinate system to a world coordinate system of a three-dimensional coordinate system is expressed as shown in the following Equation (1).
[Equation 9] ${X}_{W} = {R}^{- 1} \left({Z}_{C}{K}^{- 1}\left[\begin{matrix}{x}_{I} \\ 1\end{matrix}\right]-t\right)$ (9)

Where X_{W}=^{t}[X_{W},Y_{W},Z_{W}] represents coordinates in the world coordinate system, and x_{I}=^{t}[x_{I},y_{I}] represents coordinates in the image coordinate system. K (a 3×3 matrix) represents intrinsic parameters of an image sensor, R (a 3×3 matrix) represents a rotation matrix for coordinate transformation to the image coordinate system, t (a 3×1 vector) represents a translation vector, and Z_{C} represents depth information corresponding to two-dimensional coordinates in the image coordinate system.

These K, R, and t are calculated by camera calibration using Zhang's method ("A flexible new technique for camera calibration". IEEE Transactions on Pattern Analysis and Machine Intelligence, 22(11):1330-1334, 2000). For calibration to calculate K, a calibration board on which circular dots are printed in a grid pattern was used. Center coordinates of each dot on the calibrated board were calibrated to one-thousandth precision.

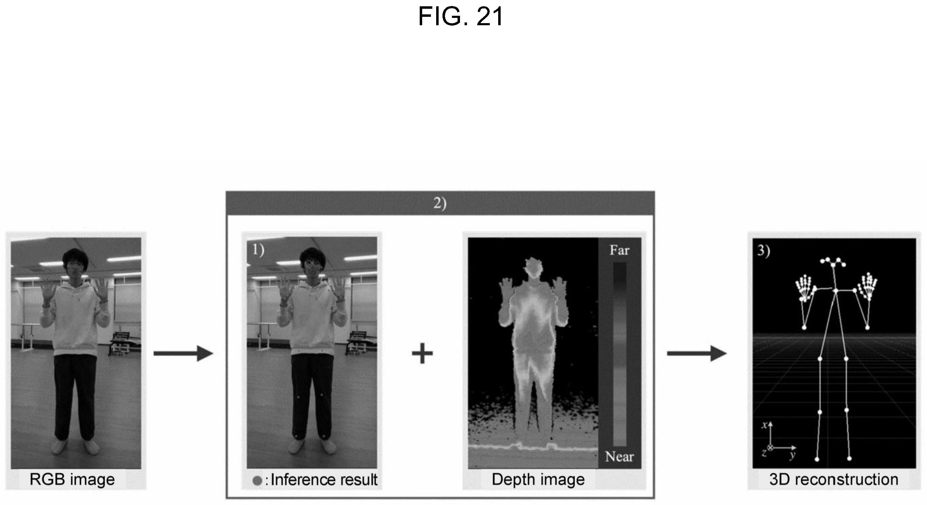

As a result, in the motion capture device 73, as shown in Figure 21, it becomes possible to acquire three-dimensional skeletal information while maintaining high robustness regardless of an imaging position by the RGB-D sensor 111, by clarifying a relationship between a position in a three-dimensional space and a position in a captured image for an entire body of the subject.

The behavior recognition unit 123 recognizes a behavior, which is a connection of a plurality of local actions, based on three-dimensional skeletal information sequentially acquired by the skeletal information acquisition unit 122 from a transition state of posture of the subject.

The behavior recognition unit 123 sequentially recognizes corresponding local actions from three-dimensional skeletal information sequentially acquired by the skeletal information acquisition unit 122 while referring to a handwashing action recognition model constructed by deep learning using action recognition patterns set for each local action as training data.

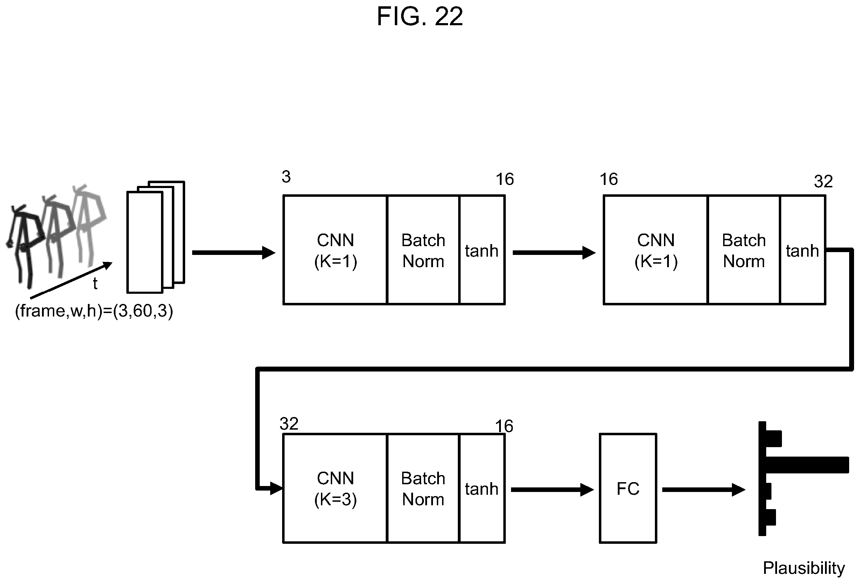

Specifically, in order to recognize a plurality of local actions, the behavior recognition unit 123 applies a behavior recognition model including three modules, namely a convolutional neural network (CNN) layer, a batch normalization layer (Batch Norm), and an activation function layer (tanh function), and a fully connected layer, as shown in Figure 22.

In the behavior recognition model, skeletal information of a current frame and a frame two frames prior thereto is input, thereby making it possible to obtain likelihoods for each of a plurality of types of local actions related to behavior.

Furthermore, as a recognition result, the behavior recognition unit 123 performs post-processing to determine, as a current local action, an action with the highest frequency among actions observed in 15 frames (a current frame and 14 preceding frames). In this manner, the behavior recognition unit 123 trained the behavior recognition model by supervised learning using an original dataset. For optimization, a cross-entropy loss function and Adam (Adaptive Moment Estimation) with a learning rate of 0.001 were used.

As a result, the motion capture device 73 can significantly improve recognition accuracy and recognition speed of local action based on three-dimensional skeletal information.

### (4) Control Adjustment According to Tensed State of Subject

In the function improvement support apparatus 1, the overall control unit 50 determines a tensed state and a relaxed state of a subject at a time of a sitting posture, a standing posture, and a transition to both postures of the subject while detecting, based on a measurement result of a normal force at contact portions with the subject (the seat portion 40 and a pair of armrest portions 30A and 30B) by the normal force measurement unit 61 (Figure 3), a transition of a center of gravity position of the subject and a load distribution to a specific portion at the contact portions.

That is, the overall control unit 50 determines a tensed state and a relaxed state of a subject by determining a current posture of the subject as a sitting posture, a standing posture, or a posture during transition to both postures, while detecting a transition of a center of gravity position of the subject and a load distribution to a specific portion (a load concentration portion in the seat portion 40 and a pair of armrest portions 30A and 30B) at contact portions of the subject, and at the same time determining to what extent the subject applies a load to the specific portion at the contact portions.

Then, the overall control unit 50 adjusts a control content of the state holding variable mechanism unit in accordance with the tensed state and the relaxed state of the subject as the determined result.

As a result, in the function improvement support apparatus 1, while a tensed state and a relaxed state are determined at a time of transition of a posture of the subject, by adjusting control relating to the mechanism switching, the transformation state, and the power application state of a state holding variable mechanism unit 2, guidance from a sitting state to a standing state of the subject and guidance from a standing state to a sitting state of the subject can be performed more safely.

### (5) Method of Detecting Environment Information

Further, in the function improvement support apparatus 1, an environment information acquisition unit 130 (Figure 3) acquires environment information representing a surrounding environment of a subject and a floor surface state on which the state holding variable mechanism unit 2 moves. Specifically, the environment information acquisition unit 130 includes a thermometer, a hygrometer, a barometer, and surrounding sound collectors for audible sound and ultrasonic sound 74, and acquires environment information of surroundings centered on a subject. This environment information mainly includes temperature, humidity, and surrounding sound in surroundings in which the subject is present.

The overall control unit 50 prioritizes ensuring safety of a subject based on environment information obtained from the environment information acquisition unit 130 when controlling the mechanism switching, the transformation state, and the power application state of the state holding variable mechanism unit 2. As a result, in the function improvement support apparatus 1, while a surrounding floor surface state and a bodily sensation state are recognized, safety of traveling and walking of the subject is ensured and a risk of falling can be significantly reduced.

### (6) Gait State Synchronized Control during Walking Assist Function

Further, in the function improvement support apparatus 1, the overall control unit 50 is configured to detect, in real time, a gait state of a subject by using the motion capture device 73 (Figure 20) described above while controlling the fourth control unit 113, and by sequentially acquiring an RGB image and a depth image of a lower body of the subject captured by an RGB-D sensor (imaging unit) 111.

That is, the image coordinate setting unit 120 sets, as image coordinates, a plurality of skeletal representative points attached to a lower body of a subject while estimating a posture of the lower body of the subject based on RGB images sequentially acquired from the RGB-D sensor (imaging unit) 111, in a state in which the posture holding variable mechanism unit 2 is transformed into the second mechanism corresponding to the wheelchair function.

Subsequently, the coordinate group extraction unit 121 sequentially extracts, from among image coordinate groups set by the image coordinate setting unit 120, image coordinate groups related to a gait state of a subject based on a transition state of posture of the subject. The skeletal information acquisition unit 122 acquires temporally continuous three-dimensional skeletal information centered on a lower body of a subject by time-synchronizing image coordinate groups sequentially extracted by the coordinate group extraction unit 121 with depth images sequentially acquired from the RGB-D sensor (imaging unit) 111.

Then, the behavior recognition unit (gait state detection unit) 123 sequentially recognizes a plurality of local actions from a transition state of posture of a subject based on three-dimensional skeletal information sequentially acquired by the skeletal information acquisition unit 122, and detects a gait state (a walking posture and a motion form of both lower limbs), which is a connection of the plurality of local actions.

The overall control unit 50 causes the front wheel drive units 43A and 43B and the rear wheel drive units 44A and 44B (wheel drive units) to generate power synchronized with a gait state detected by the behavior recognition unit (gait state detection unit) 123, and drives a pair of front wheel portions 13A and 13B and a pair of rear wheel portions 14A and 14B independently.

As a result, in the function improvement support apparatus 1, a gait state of the subject can be detected in real time, and a walking motion can be assisted while being synchronized with the gait state.

### (7) Hybrid Control during Wheelchair Function

Further, in the function improvement support apparatus 1, the overall control unit 50 is configured such that, based on muscular and skeletal system information of a subject acquired by the human information acquisition unit 62, the subject can travel without operating the controllers 46A and 46B while reflecting an operation content corresponding to an action intent of the subject.

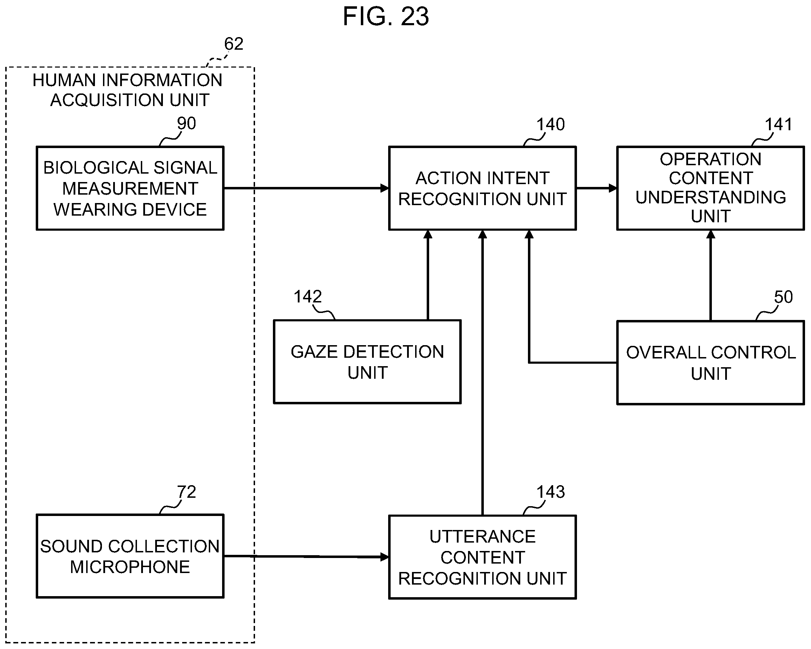

In the function improvement support apparatus 1, as shown in Figure 23, the action intent recognition unit 140, under control of the overall control unit 50, recognizes, in a state in which the posture holding variable mechanism unit 2 is transformed into the first mechanism corresponding to the wheelchair function, an action intent of a subject with respect to a specific operation content among operation contents in the wheelchair function based on a biological signal obtained from a biological signal measurement wearing device (biological signal detection unit) 90.

In practice, the action intent recognition unit 140 calculates, for each of a plurality of predetermined action intents, a likelihood (confidence level) that a signal characteristic of a biological signal matches the action intent based on the biological signal obtained from the biological signal measurement wearing device (biological signal detection unit) 90.

Specifically, the action intent recognition unit 140 obtains, by a known vector analysis method, a feature vector representing a signal characteristic of a biological signal. Since predetermined action intents each indicate a predetermined region in a vector space, the action intent recognition unit 140 calculates, in the vector space, distances between a position corresponding to a signal characteristic of a biological signal and the plurality of predetermined action intents.

Since a position in the vector space is determined by vectorization of a signal characteristic of a biological signal, the action intent recognition unit 140 calculates a distance between the position and a center of gravity position of a region of each action intent.

The action intent recognition unit 140 selects a predetermined number of positions having distances close to a signal characteristic of a biological signal in a vector space, and then, for the selected number of action intents, calculates, based on distances in the vector space, a confidence level indicating a likelihood that the signal characteristic of the biological signal matches the action intents.

The action intent recognition unit 140 calculates the confidence levels by normalizing the confidence levels such that a sum of a predetermined number of the confidence levels is 1. Further, the confidence levels may be normalized such that, in a distribution shape of the confidence levels, kurtosis (third-order moment) and skewness (fourth-order moment) take predetermined values.

Subsequently, the operation content understanding unit 141 understands, under control of the overall control unit 50, an operation content that is an action intent of a subject recognized by the action intent recognition unit 140. Specifically, the operation content understanding unit 141 stores data, as a comparison table, showing a correspondence relationship between a specific action intent and an operation content corresponding to the action intent, and selects a corresponding operation content based on the comparison table for an action intent of a subject recognized by the action intent recognition unit 140.

Then, the overall control unit 50 generates power to the front wheel drive units 43A and 43B and the rear wheel drive units 44A and 44B (wheel drive units) in accordance with an operation content understood by the operation content understanding unit 141.

As a result, in the function improvement support apparatus 1, during a wheelchair function, traveling can be performed without the subject operating the apparatus by themself while reflecting an operation content corresponding to an action intent of the subject.

### (8) Movement Control Based on Gaze Target

Further, in the function improvement support apparatus 1, a gaze detection unit 142 (Figure 23) is further provided that, while capturing an image of a space corresponding to a visual field of a subject, recognizes, by image recognition, a movement of an eyeball of the subject within an imaging range and detects a position of a gaze of the subject.

The action intent recognition unit 140 recognizes, in addition to the action intent recognition method described above, based on a position of a gaze detected by the gaze detection unit 142, that the position of the gaze is a movement target as an action intent of a subject when the position of the gaze continues for a predetermined time or longer.

As a result, in the function improvement support apparatus 1, not only a biological signal of the subject but also a position of a gaze is used to recognize an action intent of the subject with respect to an operation content, whereby traveling can be performed while reflecting an operation content corresponding to the action intent of the subject more effectively.

### (9) Voluntary Operation by Voice

Further, in the function improvement support apparatus 1, a utterance content recognition unit 143 (Figure 23) is further provided that recognizes utterance content based on a voice of a subject collected by a sound collection microphone (sound collection unit) 72.

The action intent recognition unit 140 recognizes, in addition to the action intent recognition method described above, based on utterance content recognized by the utterance content recognition unit 143, an operation content corresponding to the utterance content as an action intent of a subject.

As a result, in the function improvement support apparatus 1, not only a biological signal of the subject but also an utterance content of the subject is used to recognize an action intent of the subject with respect to an operation content, whereby traveling can be performed while reflecting an operation content corresponding to the action intent of the subject more effectively.

### (10) Other Embodiments

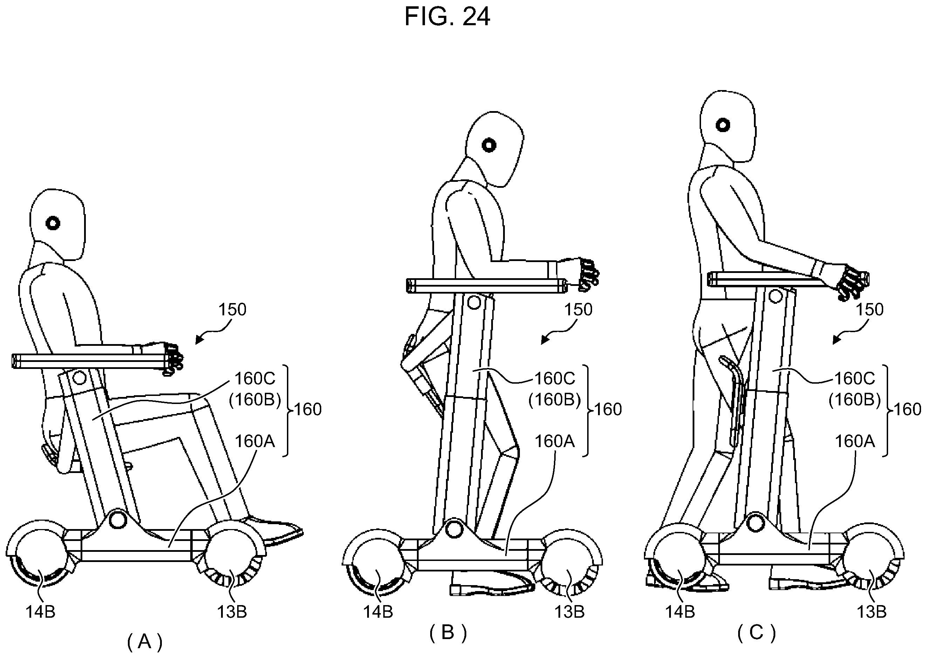

As described above, in this embodiment, although a case has been described in which the configuration of the posture holding variable mechanism unit 2 of the function improvement support apparatus 1 is as shown in Figures 1(A) and 1(B), the present invention is not limited thereto, and for example, a configuration of a posture holding variable mechanism unit 150 as shown in Figures 24(A) to 24(C) may be applied.

In Figure 24, the posture holding variable mechanism unit 150 differs from the posture holding variable mechanism unit 2 shown in Figure 1 described above in that a frame structure body 160 includes a traveling base portion 160A having a configuration in which a pair of front wheel portions 13A and 13B are bridged by a foot placement portion 11 and, together with a pair of rear wheel portions 14A and 14B, is formed in an overall substantially U-shape, and a pair of left and right support frames 160B and 160C movably supported in a front-rear direction from the traveling base portion 160A. The pair of support frames 160B and 160C are configured to be inclinable in a front-rear direction with respect to the traveling base portion 160A and to be extendable and contractible in a longitudinal direction.

As described above, in the function improvement support apparatus 1, the posture holding variable mechanism unit 150 is configured such that a first mechanism (Figure 24(A)) corresponding to a wheelchair function that holds a subject in a sitting posture and travels in accordance with an operation by the subject, and a second mechanism (Figure 24(B)) corresponding to a walking assist function that holds the subject in a standing posture and applies an assist force in accordance with a walking motion of the subject are mutually transformable while holding the subject.

Further, as described above, although, in this embodiment, a case has been described in which a task-oriented method for calculating a confidence level corresponding to an action intent of a subject is applied as a method for recognizing the action intent of the subject by the action intent recognition unit 140 (Figure 23) in the function improvement support apparatus 1, the present invention is not limited thereto, and a task-oriented method in which action intent understanding is learned may also be applied.

That is, the action intent recognition unit 140 determines an action intent from a biological signal obtained by the biological signal measurement wearing device (biological signal detection unit) 90 while referring to a recognition model constructed by deep learning using, as training data, association data in which a correspondence relationship between a signal characteristic of the biological signal and the action intent is set.

Specifically, in order to recognize a plurality of action intents, the action intent recognition unit 140 applies a recognition model including three modules, namely a convolutional neural network (CNN) layer, a batch normalization layer (Batch Norm), and an activation function layer (tanh function), and a fully connected layer. The action intent recognition unit 140 uses the recognition model to evaluate, as a degree of likelihood, whether an action intent determined based on a current state of a subject and the action intent is a desired selection result, and, based on the evaluated result, corrects association data (elimination of erroneous recognition) and learns the recognition model by supervised learning, thereby enabling recognition of the action intent of the subject with relatively high accuracy.

### Reference Signs List

1: function improvement support apparatus, 2, 150: posture holding variable mechanism unit, 10, 160: frame structure body, 11: foot placement portion, 12A: left frame, 12B: right frame, 13A, 13B: front wheel portions, 14A, 14B: rear wheel portions, 20A, 20B: front-side arm portions, 21A, 21B: rear-side arm portions, 30A, 30B: armrest portions, 31A, 31B: support arm portions, 32A, 32B: support drive units, 33A, 33B: parallel holding drive units, 40: seat portion, 40A: left seat half body, 40B: right seat half body, 41A, 41B: fixed holding portions, 42A, 42B: opening/closing drive units, 43A, 43B: front wheel drive units, 44A, 44B: rear wheel drive units, 45A, 45B: posture transformation drive units, 46A, 46B: controllers, 47A, 47B: curved frame bodies, 48A, 48B: backrest drive units, 50: overall control unit, 60: control unit, 61: normal force measurement unit, 62: human information acquisition unit, 63: drive battery, 70: vital sign measurement apparatus, 71: facial imaging camera, 72: sound collection microphone, 73: motion capture device, 74: peripheral measurement device, 75: information detection unit, 80: brain activity measurement unit, 81: blood flow measurement unit, 82: electrocardiograph, 83: first control unit, 84: wireless communication device, 90: biological signal measurement wearing device, 91: biological signal sensor, 92A to 92F: biological signal sensor group, 93: measurement module, 94: measurement module controller, 95: memory, 96: communication unit, 97: second control unit, 100: near-infrared detection unit, 101: far-infrared detection unit, 102: third control unit, 111: RGB-D sensor, 112: IMU sensor, 113: fourth control unit, 120: image coordinate setting unit, 121: coordinate group extraction unit, 122: skeletal information acquisition unit, 123: behavior recognition unit, 130: environment information acquisition unit, 140: action intent recognition unit, 141: operation content understanding unit, 142: gaze detection unit, 143: utterance content recognition unit, 160A: traveling base portion, 160B, 160C: support frames

## Claims

1. A function improvement support apparatus comprising:
a posture holding variable mechanism unit having a wheelchair function that holds a subject in a sitting posture and travels in accordance with an operation by the subject, and a walking assist function that holds the subject in a standing posture and applies an assist force in accordance with a walking motion of the subject,
the posture holding variable mechanism unit being transformable, while holding the subject, from a first mechanism corresponding to the wheelchair function to a second mechanism corresponding to the walking assist function, or from the second mechanism to the first mechanism;
a human information acquisition unit that acquires human information including brain and nervous system information, muscular and skeletal system information, physiological information, psychological information, action information, and behavior information of the subject;
a current state function determination unit that determines a current state of physical functions and cognitive functions of the subject based on the human information obtained by the human information acquisition unit;
a normal force measurement unit provided in the state holding variable mechanism unit and configured to measure, in real time, a normal force at a contact portion with the subject; and
a control unit that controls mechanism switching, a transformation state, and a power application state of the state holding variable mechanism unit based on the determination result by the current state function determination unit and the measurement result by the normal force measurement unit.

2. The function improvement support apparatus according to claim 1, wherein the control unit detects, based on the measurement result of the normal force at the contact portion with the subject by the normal force measurement unit, a transition of a center of gravity position of the subject and a load distribution to a specific portion at the contact portion, determines a tensed state and a relaxed state of the subject at a time of a sitting posture, a standing posture, and a transition to both postures of the subject while detecting the transition of the center of gravity position and the load distribution, and then adjusts a control content of the state holding variable mechanism unit in accordance with the tensed state and the relaxed state.

3. The function improvement support apparatus according to claim 1 or 2, further comprising an environment information acquisition unit that acquires environment information representing a surrounding environment of the subject and a floor surface state on which the state holding variable mechanism unit moves,
wherein the control unit, when controlling the mechanism switching, the transformation state, and the power application state of the state holding variable mechanism unit, prioritizes ensuring safety of the subject based on the environment information obtained from the environment information acquisition unit.

4. The function improvement support apparatus according to claim 1 or 2, comprising:
a wheel drive unit provided in the posture holding variable mechanism unit and configured to independently drive a pair of left and right drive wheels common to the first and second mechanisms;
an imaging unit that captures a lower body of the subject and sequentially acquires an RGB image and a depth image;
an image coordinate setting unit that, in a state in which the posture holding variable mechanism unit is transformed into the second mechanism corresponding to the wheelchair function, sets, as image coordinates, a plurality of skeletal representative points attached to a lower body of the subject while estimating a posture of the lower body of the subject based on the RGB images sequentially acquired from the imaging unit;
a coordinate group extraction unit that sequentially extracts, from among the image coordinate groups set by the image coordinate setting unit, image coordinate groups related to a gait state of the subject based on a transition state of a posture of the subject;
a skeletal information acquisition unit that acquires temporally continuous three-dimensional skeletal information centered on a lower body of the subject by time-synchronizing the image coordinate groups sequentially extracted by the coordinate group extraction unit with the depth images sequentially acquired from the imaging unit; and
a gait state detection unit that detects, based on the three-dimensional skeletal information sequentially acquired by the skeletal information acquisition unit, a gait state that is a connection of a plurality of local actions from a transition state of a posture of the subject;
wherein the control unit generates power synchronized with the gait state detected by the gait state detection unit to the wheel drive unit.

5. The function improvement support apparatus according to claim 1 or 2, comprising:
a wheel drive unit provided in the posture holding variable mechanism unit and configured to independently drive a pair of left and right drive wheels common to the first and second mechanisms;
a biological signal detection unit that is arranged on a body surface portion of the subject and has an electrode group for detecting a biological signal of the subject;
an action intent recognition unit that, in a state in which the posture holding variable mechanism unit is transformed into the first mechanism corresponding to the wheelchair function, recognizes an action intent of the subject with respect to a specific operation content among operation contents in the wheelchair function based on the biological signal obtained from the biological signal detection unit; and
an operation content understanding unit that understands an operation content that is an action intent of the subject recognized by the action intent recognition unit;
wherein the control unit generates power to the wheel drive unit in accordance with the operation content understood by the operation content understanding unit.

6. The function improvement support apparatus according to claim 5, further comprising a gaze detection unit that, while capturing an image of a space corresponding to a visual field of the subject, recognizes, by image recognition, a movement of an eyeball of the subject within an imaging range and detects a position of a gaze of the subject,
wherein the action intent recognition unit recognizes, based on the position of the gaze detected by the gaze detection unit, that the position of the gaze is a movement target as an action intent of the subject when the position of the gaze continues for a predetermined time or longer.

7. The function improvement support apparatus according to claim 5, further comprising a sound collection unit that collects a voice of the subject, and an utterance content recognition unit that recognizes an utterance content based on the voice collected by the sound collection unit,
wherein the action intent recognition unit recognizes, based on the utterance content recognized by the utterance content recognition unit, an operation content corresponding to the utterance content as an action intent of the subject.

8. A function improvement support method, in which a posture holding variable mechanism unit having a wheelchair function that holds a subject in a sitting posture and travels in accordance with an operation by the subject, and a walking assist function that holds the subject in a standing posture and applies an assist force in accordance with a walking motion of the subject, is configured to be transformable, while holding the subject, from a first mechanism corresponding to the wheelchair function to a second mechanism corresponding to the walking assist function, or from the second mechanism to the first mechanism, the method comprising:
a first step of acquiring human information including brain and nervous system information, muscular and skeletal system information, physiological information, psychological information, action information, and behavior information of the subject;
a second step of determining a current state of physical functions and cognitive functions of the subject based on the human information obtained by the first step;
a third step of measuring, in real time, a normal force at a contact portion with the subject with respect to the state holding variable mechanism unit; and
a fourth step of controlling mechanism switching, a transformation state, and a power application state of the state holding variable mechanism unit based on a determination result by the second step and a measurement result by the third step.

9. The function improvement support method according to claim 8, wherein the third step detects, based on the measurement result of the normal force at the contact portion with the subject, a transition of a center of gravity position of the subject and a load distribution to a specific portion at the contact portion, determines a tensed state and a relaxed state of the subject at a time of a sitting posture, a standing posture, and a transition to both postures of the subject while detecting the transition of the center of gravity position and the load distribution, and
the fourth step adjusts a control content of the state holding variable mechanism unit in accordance with the tensed state and the relaxed state of the subject.

10. The function improvement support method according to claim 8 or 9, further comprising a fifth step of acquiring environment information representing a surrounding environment of the subject and a road surface state on which the state holding variable mechanism unit moves,
wherein the fourth step, when controlling the mechanism switching, the transformation state, and the power application state of the state holding variable mechanism unit, prioritizes ensuring safety of the subject based on the environment information obtained from the fifth step.

11. The function improvement support apparatus according to claim 8 or 9, in which a wheel drive unit provided in the posture holding variable mechanism unit independently drives a pair of left and right drive wheels common to the first and second mechanisms, and an imaging unit captures an image of a lower body of the subject and sequentially acquires an RGB image and a depth image, the method comprising:
a sixth step of, in a state in which the posture holding variable mechanism unit is transformed into the second mechanism corresponding to the wheelchair function, setting, as image coordinates, a plurality of skeletal representative points attached to a lower body of the subject while estimating a posture of the lower body of the subject based on the RGB images sequentially acquired from the imaging unit;
a seventh step of sequentially extracting, from among the image coordinate groups set by the sixth step, image coordinate groups related to a gait state of the subject based on a transition state of a posture of the subject;
an eighth step of acquiring temporally continuous three-dimensional skeletal information centered on a lower body of the subject by time-synchronizing the image coordinate groups sequentially extracted by the seventh step with the depth images sequentially acquired from the imaging unit; and
a ninth step of detecting, based on the three-dimensional skeletal information sequentially acquired by the eighth step, a gait state that is a connection of a plurality of local actions from a transition state of a posture of the subject,
wherein the fourth step generates power synchronized with the gait state detected by the ninth step to the wheel drive unit.

12. The function improvement support method according to claim 8 or 9, in which a wheel drive unit provided in the posture holding variable mechanism unit independently drives a pair of left and right drive wheels common to the first and second mechanisms, and a biological signal of the subject is detected using an electrode group arranged on a body surface portion of the subject, the method comprising:
a tenth step of recognizing, in a state in which the posture holding variable mechanism unit is transformed into the first mechanism corresponding to the wheelchair function, an action intent of the subject with respect to a specific operation content among operation contents in the wheelchair function based on a biological signal of the subject; and
an eleventh step of understanding an operation content that is an action intent of the subject recognized by the seventh step;
wherein the fourth step generates power to the wheel drive unit in accordance with the operation content understood by the eleventh step.

13. The function improvement support method according to claim 12, further comprising a twelfth step of, while capturing an image of a space corresponding to a visual field of the subject, recognizing, by image recognition, a movement of an eyeball of the subject within an imaging range and detecting a position of a gaze of the subject,
wherein the tenth step recognizes, based on the position of the gaze detected by the twelfth step, that the position of the gaze is a movement target as an action intent of the subject when the position of the gaze continues for a predetermined time or longer.

14. The function improvement support method according to claim 12, further comprising a thirteenth step of collecting a voice of the subject and recognizing an utterance content based on the voice;
wherein the tenth step recognizes, based on the utterance content recognized by the thirteenth step, an operation content corresponding to the utterance content as an action intent of the subject.
